# EUROPEAN PATENT APPLICATION

(11) **EP 1 270 000 A2**
(43) Date of publication of application: **02.01.2003**
(21) Application number: 02254274.0
(22) Date of filing: 19.06.2002
(51) Int. Cl.: A61K 31/00, A61K 31/353, A61P 9/10

(54) **Benzoic acid substituted benzopyrans for the treatment of atherosclerosis**

(30) Priority: 28.06.2001 US 301712 P; 10.10.2001 US 328254 P
(71) Applicant: Pfizer Products Inc., Groton, Connecticut 06340 (US)
(72) Inventor: Aiello, R. J., Pfizer Global Research and Develop., Groton, Connecticut 06340 (US); Bourassa, P.-A., Pfizer Global Research and Devel., Groton, Connecticut 06340 (US); Lindsey, S., Pfizer Global Research and Developm., Groton, Connecticut 06340 (US)
(74) Representative: Sewell, Richard Charles

(57) **Abstract**

A method of treating atherosclerosis in a mammal, including a human, comprising administering to said mammal an amount of the compound of the formula 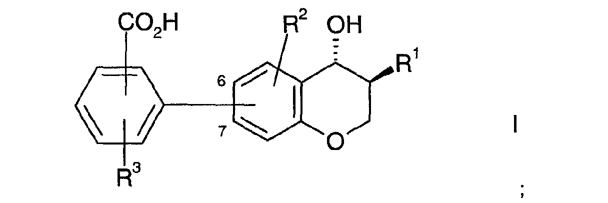 an enantiomer, or the pharmaceutically acceptable salt thereof;
effective to treat atherosclerosis, wherein R¹, R² and R³ are as defined herein.

## Description

### BACKGROUND OF THE INVENTION

This invention relates to the methods of treating atherosclerosis in a mammal, including a human, by administering an amount of LTB₄ antagonists, preferably substituted benzopyrans or a pharmaceutically acceptable salt thereof, effective in treating atherosclerosis. The substituted benzopyrans that are prepared in accord with the present invention are referred to in United States provisional patent application serial number 60/301,712, filed June 28, 2001, entitled "Benzoic Acid Substituted Benzopyrans For The Treatment of Atherosclerosis"; United States Patent numbers 5,552,435 and 6,096,906; and PCT international application publication numbers WO 96/11925, WO 96/11920, and WO 93/15066. Each of the foregoing United States and PCT international patents and patent applications are incorporated herein by reference in their entirety.

The formation of atherosclerotic lesions can occur in five overlapping stages such as migration, lipid accumulation, recruitment of inflammatory cells, proliferation of vascular smooth muscle cells, and extracellular matrix deposition. Each of these processes can occur in man and in animal models of atherosclerosis, but the relative contribution of each to the pathology and clinical significance of the lesion is unclear.

Recruitment and activation of monocytes is one aspect of a developing atherosclerotic lesion [See Gerrity, R.G., Cellular Events In Early Atherogenesis In Swine, In Swine In Biomedical Research, M.E. Tumbleson, Editor. 1985, Plenum Press: New York. p. 1497-1509]. Monocytes recruitment is governed by cell-specific chemoattractants, including an arachidonic acid metabolite leukotriene B₄ (LTB₄). LTB₄ and B₄ isoleukotrienes induce their effects by binding to a high affinity LTB₄ receptor (BLTR) [See Yokomizo T, et. al., *Nature,* 1997;387:620-624], which is expressed on inflammatory cells such as neutrophils, eosinophils and macrophages. BLTR antagonists were shown to inhibit the recruitment of neutrophils, eosinophils and macrophages in preclinical disease models, such as experimental autoimmune encephalomyditis, collagen induced arthritis and allograft transplantation [See Weringer E.J., et. al., *Transplantation*, 1999;67(6):808-815; Griffiths R, et. al., *Proc Natl Acad Sci USA*, 1995;92:517-521; and Showell H.J., et. al., *Journal of Pharmacology and Experimental Therapeutics*, 1995;273:176-184]. Thus, LTB₄ or other ligands that bind to the LTB₄ receptor may play a proatherogenic role.

The present inventors herein demonstrate that a specific LTB₄ receptor antagonist, e.g., benzoic acid substituted benzopyrans, slows lesion progression in several murine models of atherosclerosis.

### Summary of the Invention

The present invention relates to a method of treating atherosclerosis in a mammal, preferably a human, comprising administering to said mammal an amount of a compound of the formula 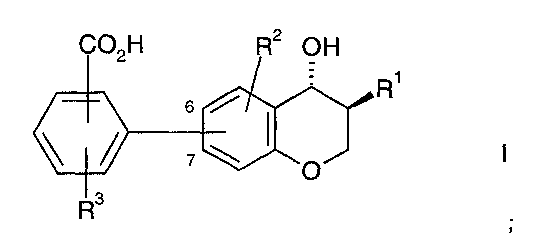 an enantiomer, a racemate, or pharmaceutically acceptable salt thereof; effective in treating atherosclerosis;
wherein in said compound of formula I the R³-substituted benzoic acid moiety is attached at carbon 6 or 7 of the benzopyran ring;
R¹ is -(CH₂)_{q}CHR⁵R⁶, wherein q is 0 to 4;
each R² and R³ is independently selected from the group consisting of hydrogen, fluoro, chloro, (C₁-C₆)alkyl (such as methyl), (C₁-C₆)alkyloxy (such as methoxy), phenylsulfinyl, phenylsulfonyl, and (C₁-C₆)alkyl-S(O)ₙ-, wherein said n is 0 to 2; wherein said R² and R³ alkyl moieties wherever they occur are independently optionally substituted by one to three fluoro groups; and wherein said R² and R³ phenyl moieties wherever they occur are independently optionally substituted by one to three fluoro groups;
R⁵ is hydrogen, (C₁-C₆)alkyl (such as methyl), or phenyl;
wherein said R⁵ phenyl is optionally substituted by one to three moieties independently selected from the group consisting of fluoro, chloro, (C₁-C₆)alkyl (such as methyl), (C₁-C₆)alkyloxy (such as methoxy), phenylsulfinyl, phenylsulfonyl, and (C₁-C₆)alkyl-S(O)ₙ-, wherein said n is 0 to 2, such as (C₁-C₆)alkylthio, (C₁-C₆)alkylsulfinyl or (C₁-C₆)alkylsulfonyl; and wherein said alkyl moieties wherever they occur on said phenyl are independently optionally substituted by one to three fluoro groups; and wherein said phenylsulfinyl and phenylsulfonyl moieties wherever they occur on said phenyl substituent are independently optionally substituted by one to three fluoro groups;
R⁶ is hydrogen, (C₁-C₆)alkyl, (C₃-C₈)cycloalkyl, phenyl, or 5 to 10 membered heteroaryl;
wherein said R⁶ phenyl is optionally substituted by one to three moieties independently selected from the group consisting of fluoro, chloro, (C₁-C₆)alkyl (such as methyl), (C₁-C₆)alkyloxy (such as methoxy), phenylsulfinyl, phenylsulfonyl, and (C₁-C₆)alkyl-S(O)ₙ-, wherein said n is 0 to 2, such as (C₁-C₆)alkylthio, (C₁-C₆)alkylsulfinyl or (C₁-C₆)alkylsulfonyl; and wherein said alkyl moieties wherever they occur on said phenyl substituent are independently optionally substituted by one to three fluoro groups; and wherein said phenylsulfinyl and phenylsulfonyl moieties wherever they occur on said phenyl substituent are independently optionally substituted by one to three fluoro groups; and
wherein said 5 to 10 membered heteroaryl is optionally substituted by one to two substituents independently selected from fluoro, chloro, (C₁-C₆)alkyl (such as methyl), (C₁-C₆)alkyloxy (such as methoxy), phenylsulfinyl, phenylsulfonyl, and (C₁-C₆)alkyl-S(O)ₙ-, wherein said n is 0 to 2, such as (C₁-C₆)alkylthio, (C₁-C₆)alkylsulfinyl or (C₁-C₆)alkylsulfonyl; wherein said alkyl moieties wherever they occur on said 5 to 10 membered heteroaryl substituent are independently optionally substituted by one to three fluoro groups; and wherein said phenylsulfinyl and phenylsulfonyl moieties wherever they occur on said 5 to 10 membered heteroaryl substituent are independently optionally substituted by one to three fluoro groups.

The term "halo", as used herein, unless otherwise indicated, means fluoro, chloro, bromo or iodo.

The term "alkyl", as used herein, unless otherwise indicated, includes saturated monovalent hydrocarbon radicals having straight, branched, or combinations thereof.

The term "alkyloxy", as used herein, includes O-alkyl groups wherein "alkyl" is defined above.

The term "aryl", as used herein, unless otherwise indicated, includes an organic radical derived form an aromatic hydrocarbon by removal of one hydrogen, such as phenyl or naphthyl.

The term "cycloalkyl", as used herein, unless otherwise indicated, refers to a mono or bicyclic carbocyclic ring (e.g., cyclopropyl, cyclobutyl, cyclopentyl, cyclohexyl, cycloheptyl, cyclooctyl, cyclononyl, cyclopentenyl, cyclohexenyl, bicyclo[2.2.1]heptanyl, bicyclo[3.2.1]octanyl and bicyclo[5.2.0]nonanyl, etc.); optionally containing 1 or 2 double bonds and optionally substituted by one to three suitable substituents as defined below such as fluoro, chloro, trifluoromethyl, (C₁₋₄)alkoxy, (C₆₋₁₀)aryloxy, trifluoromethoxy, difluoromethoxy or (C₁₋₄)alkyl, more preferably fluoro, chloro, methyl, ethyl and methoxy.

The term "heteroaryl", as used herein, unless otherwise indicated, includes an organic radical derived from an aromatic heterocyclic compound by removal of one hydrogen, such as pyridyl, furyl, thienyl, isoquinolyl, pyrimidinyl, and pyrazinyl.

The term "enantiomer", as used herein, unless otherwise indicated, includes a compound of the formula:

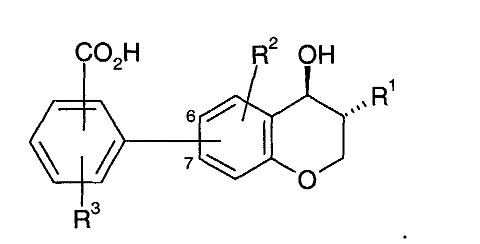

The term "racemate", as used herein, unless otherwise indicated, includes a mixture of a compound of the formula I and an enantiomer thereof, wherein the term "enantiomer" is as defined above.

The compound of formula I used in the methods of the present invention has chiral centers and is therefore exist in different enantiomeric forms. The present invention relates to all optical isomers and stereoisomers of the compounds of formula I and mixtures thereof, such as racemate thereof.

The term "sustained release" refers to a dosage form designed to release a compound of formula I for a time period ranging from about 3 to about 21 days. Release over a longer time period is also contemplated as a "sustained release" dosage form of the present invention.

The term "treating" refers to reversing, alleviating, inhibiting the progress of, or preventing the disorder or condition to which such term applies, or one or more symptoms of such disorder or condition. The term "treatment", as used herein, refers to the act of treating, as "treating" is defined immediately above.

The term "LTB₄ receptor antagonists" refers to compounds of formula I, as defined above.

The term "atherosclerosis" refers to the formation of an atherosclerotic lesion in five overlapping stages such as migration, lipid accumulation, recruitment of inflammatory cells, proliferation of vascular smooth muscle cells, and extracellular matrix deposition.

The term "LTB₄ antagonist agent" as used herein refers to any chemical or pharmaceutical molecule that possesses the claimed activity against LTB₄ as measured by standard assays (such as chemotaxis assay or direct binding assay), such as DNA, RNA, antisense or sense oligonucleotide, monoclonal or polyclonal antibody or small molecule.

The term "small molecule", as used herein, refers to non-DNA, non-RNA, non-polypeptide, and non-monoclonal antibody molecules with a molecular weight of under 2000 grams/mole, preferably under 500 grams/mole. Preferred small molecules contain aromatic rings (such as aryl or heteroaryl). More preferred small molecules contain phenyl or benzopyranyl group. Most preferred small molecules are phenothiazin-3-one class of compounds to which L-651,392 of Merck belongs; the class of amidino compounds to which CGS-25019 of Novartis belongs; the class of benzoxaolamines to which ontazolast belongs; the class of benzenecarboximidamides to which BIIL 284/260 of Boehringer belongs; the class of 2-(substituted)-N-hydroxy-N-alkylcinnamamides to which LY-233,569 of Lilly belongs; the class of compounds to which ebselen belongs; the class of compounds to which linazolast belongs; the class of compounds to which Bay-x-1005 of Bayer belongs; the class of compounds to which ETH-615 of Leo Denmark belongs; the class of compounds to which MAFP of Merck belongs; the class of compounds to which TMK-688 of Terumo belongs; the class of compounds to which T-0757 of Tanabe belongs; the class of compounds to which LY-213024, LY-210073, LY-223982, LY-233469, LY-255283, LY-293111, LY-264086 and LY-292728 of Lilly belong; the class of compounds to which ONO-LB457, ONO-4057, and ONO-LB448 of ONO belong; the class of compounds to which S-2474 of Shionogi belongs; the class of compounds to which calcitrol belongs; the class of compounds to which SC-53228, SC-41930, SC-50605 and SC-51146 of Searle belongs; the class of compounds to which BPC-15 of Warner Lambert belongs; the class of compounds to which SB-209247 of SmithKline Beecham belongs; or the class of compounds to which SKF-104493 of SK&F belongs.

The term "lesion", as used herein, is as described in Ross, R. THE PATHOGENESIS OF ATHEROSCLEROSIS IN HEART DISEASE 1106-1124 (E. Braunwald ed., W.B. Saunders Company 1992), herein incorporated by reference.

The term "plaque" or "plaque stability", as used herein, is as described in Ross, R. THE PATHOGENESIS OF ATHEROSCLEROSIS IN HEART DISEASE 1106-1124 (E. Braunwald ed., W.B. Saunders Company 1992), herein incorporated by reference.

The term "LTB₄ IC₅₀ of less than about 20 nM, preferably less than about 10 nM, as measured by chemotaxis assay", as used herein, is as described in Doherty et. al., *The In Vitro and In Vivo Pharmacologic Activity of the Potent and Selective Leukotriene B4 Receptor Antagonist CP-105,696*, J. Pharm. and Exp. Ther, 273:176-184 (1995); and Showell et. al., *Characterization of The Pharmacological Profile of The Potent LTB*_{*4*} *Antagonist CP-105,696 on Murine LTB*_{*4*} *Receptors In Vitro*, Br. J. Pharmacol., 117:1127-1132 (1996), herein incorporated by reference.

In a preferred embodiment of the invention, the compound of formula I inhibits progression of lipid lesions. As exemplified hereinbelow, benzoic acid substituted benzopyran inhibits the progression of lipid lesions, in a mammal.

In a preferred embodiment of the invention, R¹ in said compound of formula I is benzyl, 4-fluorobenzyl, 4-phenylbenzyl, 4-(4-fluorophenyl)benzyl, or phenethyl.

In a preferred embodiment of the invention, R² in said compound of formula I is hydrogen or fluoro.

In a preferred embodiment of the invention, said R³-substituted benzoic acid moiety in said compound of formula I is attached at carbon 7 of said benzopyran ring; and wherein said R³-substituted benzoic acid moiety is 2-carboxyphenyl, 2-carboxy-5-chlorophenyl, 2-carboxy-4-chlorophenyl, 2-carboxy-3-fluorophenyl, 2-carboxy-5-fluorophenyl, 2-carboxy-5-trifluoromethylphenyl, 2-carboxy-4-fluorophenyl, 2-carboxy-6-fluorophenyl, or 3-carboxyphenyl.

In a preferred embodiment of the invention, R¹ in said compound of formula I is benzyl, R² is hydrogen, and said R³-substituted benzoic acid moiety is 2-carboxy-5-fluorophenyl.

In a preferred embodiment of the invention, R¹ in said compound of formula I is 4-phenylbenzyl, R² is hydrogen, and said R³-substituted benzoic acid moiety is 2-carboxy-5-fluorophenyl or 2-carboxy-4-chlorophenyl.

In a specific preferred embodiment of the invention, said compound of formula I is selected from the group consisting of
(3S,4R)-7-(2-carboxyphenyl)-4-hydroxy-3-benzyl-3,4-dihydro-2H-1 -benzopyran;
(3S,4R)-7-(2-carboxy-5-chlorophenyl)-4-hydroxy-3-benzyl-3,4-dihydro-2H-1-benzopyran;
(3S,4R)-7-(2-carboxy-4-chlorophenyl)-4-hydroxy-3-benzyl-3,4-dihydro-2H-1-benzopyran;
(3S,4R)-7-(2-carboxy-3-fluorophenyl)-4-hydroxy-3-benzyl-3,4-dihydro-2H-1-benzopyran;
(3S,4R)-7-(2-carboxy-4-fluorophenyl)-4-hydroxy-3-benzyl-3,4-dihydro-2H-1-benzopyran;
(3S,4R)-7-(2-carboxy-5-fluorophenyl)-4-hydroxy-3-benzyl-3,4-dihydro-2H-1-benzopyran;
(3S,4R)-7-(2-carboxy-5-trifluoromethylphenyl)-4-hydroxy-3-benzyl-3,4-dihydro-2H-1-benzopyran; and
(3S,4R)-7-(3-carboxyphenyl)-4-hydroxy-3-benzyl-3,4-dihydro-2H-1-benzopyran.

In a most preferred embodiment of the invention, said compound of formula I is (3S, 4R)-7-(2-carboxy-5-trifluoromethylphenyl)-4-hydroxy-3-benzyl-3,4-dihydro-2H-1-benzopyran.

In one embodiment of the foregoing, said amount of the compound of formula I is 0.5 to 1000 mg/day.

In one embodiment of the foregoing, the compound of formula I is administered orally.

In a further embodiment of the foregoing, the compound of formula I is impregnated in a stent, such as intravascular stent, which may be metallic, plastic or a biodegradable polymer. For a general discussion of implantable devices and biomaterials from which they can be formed, see H. Kambic et al., "Biomaterials in Artificial Organs", *Chem. Eng. News*, 30 (1986), which is incorporated by reference herein.

In a further embodiment of the foregoing, the compound of formula I impregnated in said stent, such as intravascular stent, is (3S, 4R)-7-(2-carboxy-5-trifluoromethylphenyl)-4-hydroxy-3-benzyl-3,4-dihydro-2H-1-benzopyran.

A stent useful in the present invention can comprise a biodegradable coating or porous non-biodegradable coating, having dispersed therein a sustained-release dosage form. In an alternative embodiment, a biodegradable stent may also have a compound of formula I impregnated therein, *i.e.*, in a stent matrix. Utilization of a biodegradable stent with a compound of formula I impregnated therein is further coated with a biodegradable coating or with a porous non-biodegradable coating having a sustained release-dosage form dispersed therein is also contemplated. This embodiment of the invention would provide a differential release rate of the compound of formula I, *i.e.* there would be a faster release of the compound of formula I from the coating followed by delayed release of the compound of formula I that was impregnated in a stent matrix upon degradation of the stent matrix.

In a further embodiment of the foregoing, the compound of formula I impregnated in said stent, such as intravascular stent, wherein said stent is further coated with a biodegradable coating or with a porous non-biodegradable coating having a sustained release-dosage form of said compound of formula I dispersed therein.

The present invention also relates to a method of treating atherosclerosis in a mammalian subject having an atherosclerotic lesion, comprising administering to said subject a therapeutically effective amount of an LTB₄ antagonist agent, preferably a small molecule, more preferably a small molecule containing phenyl or benzopyranyl group, that effectively reduces the progression of formation of said lesion.

In one embodiment, the present invention relates to a method of treating atherosclerosis, in a mammalian subject, preferably a human subject, comprising administering to said subject having an atherosclerotic lesion a therapeutically effective amount of an LTB₄ antagonist agent, preferably a small molecule, more preferably a small molecule containing phenyl or benzopyranyl group, that effectively improves plague stability by improving plaque stability.

In another embodiment, the present invention relates to a method of treating atherosclerosis, in a mammalian subject, preferably a human subject, comprising administering to said subject having an atherosclerotic lesion a therapeutically effective amount of an LTB₄ antagonist agent, wherein said agent is selected from the group consisting of an antibody for LTB₄ receptor and an antisense for LTB₄ gene.

In another embodiment, the present invention relates to a method of treating atherosclerosis, in a mammalian subject, preferably a human subject, comprising administering to said subject having an atherosclerotic lesion a therapeutically effective amount of an LTB₄ antagonist agent, wherein said LTB₄ antagonist agent exhibits an LTB₄ IC₅₀ of less than about 20 nM, preferably of less than about 10 nM, as measured by chemotaxis assay.

In still another embodiment, the present invention relates to a method of treating atherosclerosis, in a mammalian subject, preferably a human subject, comprising administering to said subject having an atherosclerotic lesion a therapeutically effective amount of a small molecule, wherein said small molecule exhibits an LTB₄ IC₅₀ of less than about 20 nM, preferably less than about 10 nM, as measured by chemotaxis assay.

In still another embodiment, the present invention relates to a method of treating an atherosclerotic lesion in a mammalian subject, comprising administering to said subject a composition comprising an amount of an LTB₄ antagonist agent, preferably a small molecule, that effectively reduces the progression of formation of said lesion.

In a further embodiment of the foregoing, the compound of formula I, preferably (3S, 4R)-7-(2-carboxy-5-trifluoromethylphenyl)-4-hydroxy-3-benzyl-3,4-dihydro-2H-1-benzopyran, is administered in combination with one or more members selected from the group consisting of the following: (a) leukotriene biosynthesis inhibitors: 5-lipoxygenase (5-LO) inhibitors and 5-lipoxygenase activating protein (FLAP) antagonists selected from the group consisting of zileuton; ABT-761; fenleuton; tepoxalin; Abbott-79175; Abbott-85761; *N*-(5-substituted)-thiophene-2-alkylsulfonamides; 2,6-di-*tert*-butylphenol hydrazones; a class of methoxytetrahydropyrans which includes Zeneca ZD-2138; compound SB-210661 and the class to which it belongs; the class of pyridinyl-substituted 2-cyanonaphthalene compounds to which L-739,010 belongs; the class of 2-cyanoquinoline compounds to which L-746,530 belongs; the classes of indole and quinoline compounds to which MK-591, MK-886, and BAY x 1005 belong; (b) receptor antagonists for leukotrienes LTB₄, LTC₄, LTD₄, and LTE₄ selected from the group consisting of the phenothiazin-3-one class of compounds to which L-651,392 belongs; the class of amidino compounds to which CGS-25019 belongs; the class of benzoxaolamines to which ontazolast belongs; the class of benzenecarboximidamides to which BIIL 284/260 belongs; and the classes of compounds to which zafirlukast, ablukast, montelukast, pranlukast, verlukast (MK-679), RG-12525, Ro-245913, iralukast (CGP 45715A), and BAY x 7195 belong; (c) PDE4 inhibitors including inhibitors of the isoform PDE4D; (d) 5-Lipoxygenase (5-LO) inhibitors; or 5-lipoxygenase activating protein (FLAP) antagonists; (e) dual inhibitors of 5-lipoxygenase (5-LO) and antagonists of platelet activating factor (PAF); (f) leukotriene antagonists (LTRAs) including antagonists of LTB₄, LTC₄, LTD₄, and LTE₄; (g) antihistaminic H₁ receptor antagonists including cetirizine, loratadine, desloratadine, fexofenadine, astemizole, azelastine, and chlorpheniramine; (h) gastroprotective H₂ receptor antagonists; (i) α₁- and α₂-adrenoceptor agonist vasoconstrictor sympathomimetic agents administered orally or topically for decongestant use, including propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride; (j) α₁- and α₂-adrenoceptor agonists in combination with inhibitors of 5-lipoxygenase (5-LO); (k) anticholinergic agents including ipratropium bromide; tiotropium bromide; oxitropium bromide; pirenzepine; and telenzepine; (I) β₁- to β₄-adrenoceptor agonists including metaproterenol, isoproterenol, isoprenaline, albuterol, salbutamol, formoterol, salmeterol, terbutaline, orciprenaline, bitolterol mesylate, and pirbuterol; (m) methylxanthanines including theophylline and aminophylline; (n) sodium cromoglycate; (o) muscarinic receptor (M1, M2, and M3) antagonists; (p) COX-1 inhibitors (NSAIDs); COX-2 selective inhibitors including rofecoxib; and nitric oxide NSAIDs; (q) insulin-like growth factor type I (IGF-1) mimetics; (r) ciclesonide; (s) inhaled glucocorticoids with reduced systemic side effects, including prednisone, prednisolone, flunisolide, triamcinolone acetonide, beclomethasone dipropionate, budesonide, fluticasone propionate, and mometasone furoate; (t) tryptase inhibitors; (u) platelet activating factor (PAF) antagonists; (v) monoclonal antibodies active against endogenous inflammatory entities; (w) IPL 576; (x) antitumor necrosis factor (TNFα) agents including Etanercept, Infliximab, and D2E7; (y) DMARDs including Leflunomide; (z) TCR peptides; (aa) interleukin converting enzyme (ICE) inhibitors; (bb) IMPDH inhibitors; (cc) adhesion molecule inhibitors including VLA-4 antagonists; (dd) cathepsins; (ee) MAP kinase inhibitors; (ff) glucose-6 phosphate dehydrogenase inhibitors; (gg) kinin-B₁ - and B₂-receptor antagonists; (hh) gold in the form of an aurothio group together with various hydrophilic groups; (ii) immunosuppressive agents, *e.g.*, cyclosporine, azathioprine, and methotrexate; (jj) anti-gout agents, *e.g.*, colchicine; (kk) xanthine oxidase inhibitors, *e.g.*, allopurinol; (II) uricosuric agents, *e.g.*, probenecid, sulfinpyrazone, and benzbromarone; (mm) antineoplastic agents, especially antimitotic drugs including the vinca alkaloids such as vinblastine and vincristine; (nn) growth hormone secretagogues; (oo) inhibitors of matrix metalloproteases (MMPs), *i.e.*, the stromelysins, the collagenases, and the gelatinases, as well as aggrecanase; especially collagenase-1 (MMP-1), collagenase-2 (MMP-8), collagenase-3 (MMP-13), stromelysin-1 (MMP-3), stromelysin-2 (MMP-10), and stromelysin-3 (MMP-11); (pp) transforming growth factor (TGFβ); (qq) platelet-derived growth factor (PDGF); (rr) fibroblast growth factor, *e.g.*, basic fibroblast growth factor (bFGF); (ss) granulocyte macrophage colony stimulating factor (GM-CSF); (tt) capsaicin cream; (uu) Tachykinin NK₁ and NK₃ receptor antagonists selected from the group consisting of NKP-608C; SB-233412 (talnetant); and D-4418; (w) elastase inhibitors selected from the group consisting of UT-77 and ZD-0892; (ww) HMG CoA Reductase Inhibitors such as Lovastatin; (xx) Lipitor; (yy) Fibric Acids such as ciprofibrate and clofibrate; (zz) Bile Acid Binding Agents such as cholestyramine and colestipol; (aaa) Nicotinic Acid; and (bbb) Lipophilic anti-oxidants such as Probucol.

In a further embodiment of the foregoing, the compound of formula I is administered as prodrugs of compounds of the formula I. Compound of formula I having free amino, amido, hydroxy or carboxylic groups can be converted into prodrugs. Prodrugs include compounds wherein an amino acid residue, or a polypeptide chain of two or more (*e.g.*, two, three or four) amino acid residues which are covalently joined through peptide bonds to free amino, hydroxy or carboxylic acid groups of compound of formula I. The amino acid residues include the 20 naturally occurring amino acids commonly designated by three letter symbols and also include 4-hydroxyproline, hydroxylysine, demosine, isodemosine, 3-methylhistidine, norvalin, beta-alanine, gamma-aminobutyric acid, citrulline, homocysteine, homoserine, ornithine and methionine sulfone. Prodrugs also include compounds wherein carbonates, carbamates, amides and alkyl esters, which are covalently bonded to the above substituents of formula I through the carbonyl carbon prodrug sidechain.

### Detailed Description of the Invention

For administration to mammals, including humans, of LTB₄ receptor antagonists, suitably a benzoic acid substituted benzopyran, of the present methods of treating atherosclerosis, a variety of conventional routes may be used. Suitable routes include oral, parenteral (*e.g.*, intravenous, intramuscular, intraperitoneal, or subcutaneous), buccal, rectal, intranasal, and transdermal. In general, the compounds of the invention (hereinafter also known as the active compounds) may be administered at dosages between about 0.5 to 1000 mg/day.

Preferably the active compound will be administered orally. However, some variation in dosage will necessarily occur depending on the condition of the subject being treated. The person responsible for administration will, in any event, determine the appropriate dose for the individual subject.

The active compounds can be administered in a wide variety of different dosage forms, in general, the effective amount of the compounds of this invention are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelation and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof. In the case of animals, they are advantageously contained in an animal feed or drinking water in a concentration of 5-5000 ppm, preferably 25 to 500 ppm.

For parenteral administration (intravenous, intramuscular, intraperitoneal, or subcutaneous use) a sterile injectable solution of the active ingredient is usually prepared. Solutions of a compound of the present invention in either sesame or peanut oil or in aqueous propylene glycol may be employed. The aqueous solutions should be suitably adjusted and buffered, preferably at a pH of greater than 8, if necessary and the liquid diluent first rendered isotonic. These aqueous solutions are suitable intravenous injection purposes. The oily solutions are suitable for intravenous, intramuscular and subcutaneous injection purposes. The preparation of all these solutions under sterile conditions is readily accomplished by standard pharmaceutical techniques well known to those skilled in the art. In the case of animals, compounds can be administered intramuscularly or subcutaneously at dosage levels of about 0.1 to 50 mg/kg/day, advantageously 0.2 to 10 mg/kg/day given in a single dose or up to 3 divided doses.

For buccal administration, the composition may take the form of tablets or lozenges formulated in conventional manner.

For rectal administration, the active compounds of the invention may also be formulated in rectal compositions such as suppositories or retention enemas, e.g., containing conventional suppository bases such as cocoa butter or other glycerides.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant, e.g., dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the active compound. Capsules and cartridges (made, for example, from gelatin) for use in an inhaler or insufflator may be formulated containing a powder mix of a compound of the invention and a suitable powder base such as lactose or starch.

For transdermal administration, transdermal patches prepared in accordance with well known drug delivery technology may be prepared and applied to the skin of a mammal, preferably a human or a dog, to be treated, whereafter the active agent by reason of its formulated solubility characteristics migrates across the epidermis and into the dermal layers of the skin where it is taken up as part of the general circulation, ultimately providing systemic distribution of the active ingredient over a desired, extended period of time. Also included are implants which are placed beneath the epidermal layer of the skin, *i.e.* between the epidermis and the dermis of the skin of the patient being treated. Such an implant will be formulated in accordance with well known principles and materials commonly used in this delivery technology, and may be prepared in such a way as to provide controlled-, sustained-, and/or delayed-release of the active ingredient into the systemic circulation of the patient. Such subepidermal (subcuticular) implants provide the same facility of installation and delivery efficiency as transdermal patches, but without the limitation of being subject to degradation, damage or accidental removal as a consequence of being exposed on the top layer of the patient's skin.

Experimentation conducted to ascertain the impact of compound of formula I, preferably (3S, 4R)-7-(2-carboxy-5-trifluoromethylphenyl)-4-hydroxy-3-benzyl-3,4-dihydro-2H-1-benzopyran, on atherosclerosis in mammals, specifically mice, is set forth in detail in the Examples section below.

The present invention also contemplates methods and dosage forms involving sustained release of an LTB₄ receptor antagonist to target cells. Suitably, the target cells are vascular smooth muscle cells, cancer cells, somatic cells requiring modulation to ameliorate a disease state and cells involved in immune system-mediated diseases that are accessible by local administration of the dosage form. Consequently, the methods and dosage forms of this aspect of the present invention are useful for inhibiting vascular smooth muscle cells in a mammalian host, employing an LTB₄ receptor antagonist that inhibits the activity of the cell (e.g., migration, proliferation, formation of lipid proliferative lesions, contraction, or the like) but does not kill the cell and, optionally, a vascular smooth muscle cell binding protein. Sustained released dosage forms can also be employed in the practice of the present method.

Formulations intended for the controlled release of pharmaceutically-active compounds in vivo include solid particles of the active ingredient that are coated or tableted with film-forming polymers, waxes, fats, silica, and the like. These substances are intended to inhibit the dissolution, dispersion or absorption of the active ingredient in vivo. Hydroxypropylmethyl cellulose is one example of an ingredient that can provide a slow or controlled release of the active ingredient. The compounds can also be delivered via patches for transdermal delivery, subcutaneous implants, infusion pumps or via release from implanted sustained release dosage forms.

Another embodiment of the present methods of the invention relates to sustained release methods from the surface of an intravascular device, such as a stent, employing an excipient matrix which can release an LTB₄ receptor antagonist of the present invention over a one-week to two-year or longer period. The surface coating and the impregnated forms of the article can be a biodegradable or nonbiodegradable polymer or ceramic material which can slowly release the LTB₄ receptor antagonists of the present invention at a dose rate that can inhibit the proliferation of fibromuscular cells and/or lipid accumulation which would impair the function of the device. The accumulation of fibromuscular cells, and their associated matrix, along with lipid containing foam cells can decrease the lumenal area of a stent to an extent that blood flow is critically impaired and the device can fail functionally. The inhibition of this proliferation would extend the clinically functional life of the stent and be of clinical benefit to the patients.

The sustained release dosage forms of this embodiment of the invention needs to deliver a sufficient anti-proliferative, suitably cytostatic, dosage to expose cells immediately adjacent to the device surface to treat atherosclerosis. This would inhibit cellular attachment, migration and proliferation of the fibromuscular cells and foam cells. This dosage is determinable empirically by implanting a specific device intravascularly with variable amounts of an LTB₄ receptor antagonist of the invention and modification of the polymer excipient, both of which would affect the rate and duration of the drug release required to achieve the cytostatic dosing. Stent is considered a permanently implanted device; however, it may not be necessary to have the active agent continuously released from the device. It appears from initial observations that if excessive proliferation is prevented until the stent is surrounded by quiescent tissue and covered by intact endothelium then continued release of the LTB₄ receptor antagonists may be unnecessary.

The sustained release dosage forms of the present invention are suitably either nondegradable microparticulates or nanoparticulates or biodegradable microparticulates or nanoparticulates. More suitably, the microparticles or nanoparticles are formed of a polymer containing matrix that biodegrades by random, nonenzymatic, hydrolytic scissioning. A suitable structure is formed of a mixture of thermoplastic polyesters (e.g., polylactide or polyglycolide) or a copolymer of lactide and glycolide components. The lactide/glycolide structure has an added advantage that biodegradation thereof forms lactic acid and glycolic acid, both normal metabolic products of mammals.

The sustained release dosage forms of the present invention exhibit the capability to deliver an LTB₄ receptor antagonist to target cells over a sustained period of time. Such dosage forms of the present invention may be of any configuration suitable for this purpose. Suitable sustained release dosage forms exhibit one or more of the following characteristics:
1. microparticulate (e.g., from about 0.5 micrometers to about 100 micrometers in diameter, suitably from about 0.5 to about 2 micrometers) or nanoparticulate (e.g., from about 1.0 nanometer to about 1000 nanometers in diameter, suitably from about 50 to about 250 nanometers), free flowing powder structure;
2. biodegradable structure designed to biodegrade over a period of time between from about 3 to about 180 days, suitably from about 10 to about 21 days, or nonbiodegradable structure to allow a diffusion of the LTB₄ receptor antagonist to occur over a time period of between from about 3 to about 180 days, suitably from about 10 to about 21 days;
3. biocompatible with target tissue and the local physiological environment into which the dosage form is being administered, including biocompatible biodegradation products;
4. facilitate a stable and reproducible dispersion of the LTB₄ receptor antagonists therein, suitably to form a LTB₄ receptor antagonist-polymer matrix, with the LTB₄ receptor antagonist release occurring through one or both of the following routes: (1) diffusion of the LTB₄ receptor antagonist through the dosage form (when the LTB₄ receptor antagonist is soluble in the polymer or polymer mixture forming the dosage form); or (2) release of the LTB₄ receptor antagonist as the dosage form biodegrades; and capability to bind with one or more cellular and/or interstitial matrix epitopes, suitably from about 1 to about 10,000 binding protein/peptide-dosage form bonds, and more suitably having a maximum of about 1 binding peptide-dosage form per 150 square angstroms of particle surface area. The total number bound depends upon the particle size used. The binding proteins or peptides are capable of coupling to the particulate dosage form through covalent ligand sandwich or non-covalent modalities as set forth herein.

For example, nanoparticles containing a compound of the formula I may be prepared using biodegradable polymers including poly(D,L-lactic acid)PLA, poly(D,L-lactic-co-glycolic) PLGA, methacrylic acid copolymer, poly(epsilon-caprolactone), using either 1) n-solvent emulsification-evaporation techniques or 2) emulsification - precipitation techniques. These processes involve dispersion of polymer in an organic solvent (e.g., acetone or benzyl alcohol) with or without a co-solvent, typically methylene chloride. The compound of formula I is contained in the organic solvent. In some cases, solvents are then mixed and then added dropwise to an aqueous solution containing stabilizing hydrocolloid [e.g., poly(vinyl alcohol) or gelatin] (i.e., oil in water) with mechanical agitation or sonication. Following formation of the stable emulsion, the chlorinated solvent is removed via evaporation of the stirred emulsion, yielding nanoparticles that then can be freed of organic solvents by tangential filtration or repeated washings by centrifugation/resuspension. The resultant aqueous suspension can then be frozen with or without saccharide or other cryoprotectants and lyophilized to yield nanoparticles capable of resuspension in physiological salt solutions with simple agitation or sonication.

Alternatively, the aqueous solution can be added with agitation or sonication to the organic phase lacking chlorinated solvent (i.e., water-in-oil emulsion) followed by further addition of aqueous solution to achieve a phase inversion, to precipitate the nanoparticles. Alternatively, precipitation can be augmented by addition to salting-out agents in the aqueous solvent. Typically, for emulsification-evaporation technique 750 mg PLGA can be dissolved in 30 ml of methylene chloride. Suitably, 5 ml of methylene chloride containing 75 mg of a compound of formula I is added. This organic phase is added dropwise to 180 ml of aqueous solution of 2.5% poly(vinyl alcohol, PVP) (20-70 kD mol Wt.) with sonication using a sonifier at 15-55 watt output, for approximately 10 minutes to form a soluble emulsion. Sonication is performed in an ice bath at a temperature not exceeding 15°C. The emulsion is then further stirred at room temperature for 24 hours to allow for evaporation of the chlorinated solvent. The resultant nanoparticles are purified further a filtration device fitted with a 100 mm pore polyolefin cartridge filter. For the emulsification-precipitation technique, 10 µL of aqueous PMP (10-30% w/w) is added, under mechanical stirring at 1200-5000 rpm, to 5 mL of benzyl alcohol containing 10-15% w/w polymer PLA or PLGA and 10-15 w/w of a compound of the formula I, following oil-in-water emulsion formation over 5 minutes. Water (160 mL) is then added to effect a phase inversion, resulting in diffusion of organic solvent into the water with concomitant precipitation of polymer as solid nanoparticles in the ensuing 10 minutes.

The formation of the atherosclerotic lesion can occur in five stages:
1. MIGRATION. In a healthy vessel, most or all of the smooth muscle cells (SMC) are contained in vessel media. The appearance of SMC in an enlarged intima during lesion formation must therefore require migration of the SMC from the media to the intima of the vessel. Inhibition of this SMC migration would significantly alter the nature of a lesion, and may ameliorate the pathology associated with lesion formation.
2. LIPID ACCUMULATION. Medial SMC in healthy vessel walls do not significantly accumulate lipid. However, intimal SMC have an increased capacity for lipid uptake and storage. When exposed to elevated levels of circulating lipid (particularly low density lipoprotein; LDL), SMC may become saturated with fatty lipid and die. The accumulation of lipid is necessary for the progression of the lesion to clinical significance, since it forms the thrombogenic necrotic core of the lesion. Inhibition of lipid accumulation in the SMC should reduce or prevent lesion progression, thus reducing or preventing atherosclerosis and resultant myocardial infarction.
3. RECRUITMENT OF INFLAMMATORY CELLS. Human lesions contain many macrophage-derived cells. The process of recruitment, the function of these cells, and their contribution to pathology are unclear. An oversimplified mechanism suggests that macrophages are attracted to the lipid accumulating in the lesion, in order to remove the lipid from the vessel wall. While inhibition of recruitment of macrophage-derived cells might reduce lesion pathology, it may also speed progression to the lipid-filled, rupture-prone state.
4. PROLIFERATION. Intimal SMC accumulation is accompanied by medial thinning in many cases. Therefore, total SMC number may not increase significantly at the lesion site. Furthermore, the chronic nature of atherosclerosis makes it difficult to detect stimulation of proliferation in these lesions.
5. EXTRACELLULAR MATRIX DEPOSITION. Atherosclerotic lesions are also rich in extracellular matrix (ECM), and in particular, collagen fibers. Increased ECM synthesis may increase plaque stability. Early plaque rupture, leading to myocardial infarction, may be associated with low ECM deposition and resultant weakening of the fibrous cap that overlays the necrotic, lipid-rich core of the lesion.

Accordingly, atherosclerosis involves the complex interplay of various processes, some of which may be yet unidentified. Targeting a single process in an effort to treat atherosclerosis depends on knowledge of the relative contribution of each process to the manifested pathology. For these reasons, a coordinated strategy is suitable. An exemplary strategy involves inhibition of SMC migration, lipid accumulation and proliferation, with possible beneficial effects of increasing ECM deposition.

The compound of formula I used in the present methods of treating atherosclerosis may be prepared by methods as illustrated in the following Schemes. In the following Schemes and discussion that follows, unless otherwise indicated, R¹, R², R³, R⁵, and R⁶ are as defined above. The following Schemes and the discussion that follows describe the preparation of the compounds of formulas I-XIX. The following Schemes and description that follows also applies to the enantiomers of the compounds of formulas I-XIX.

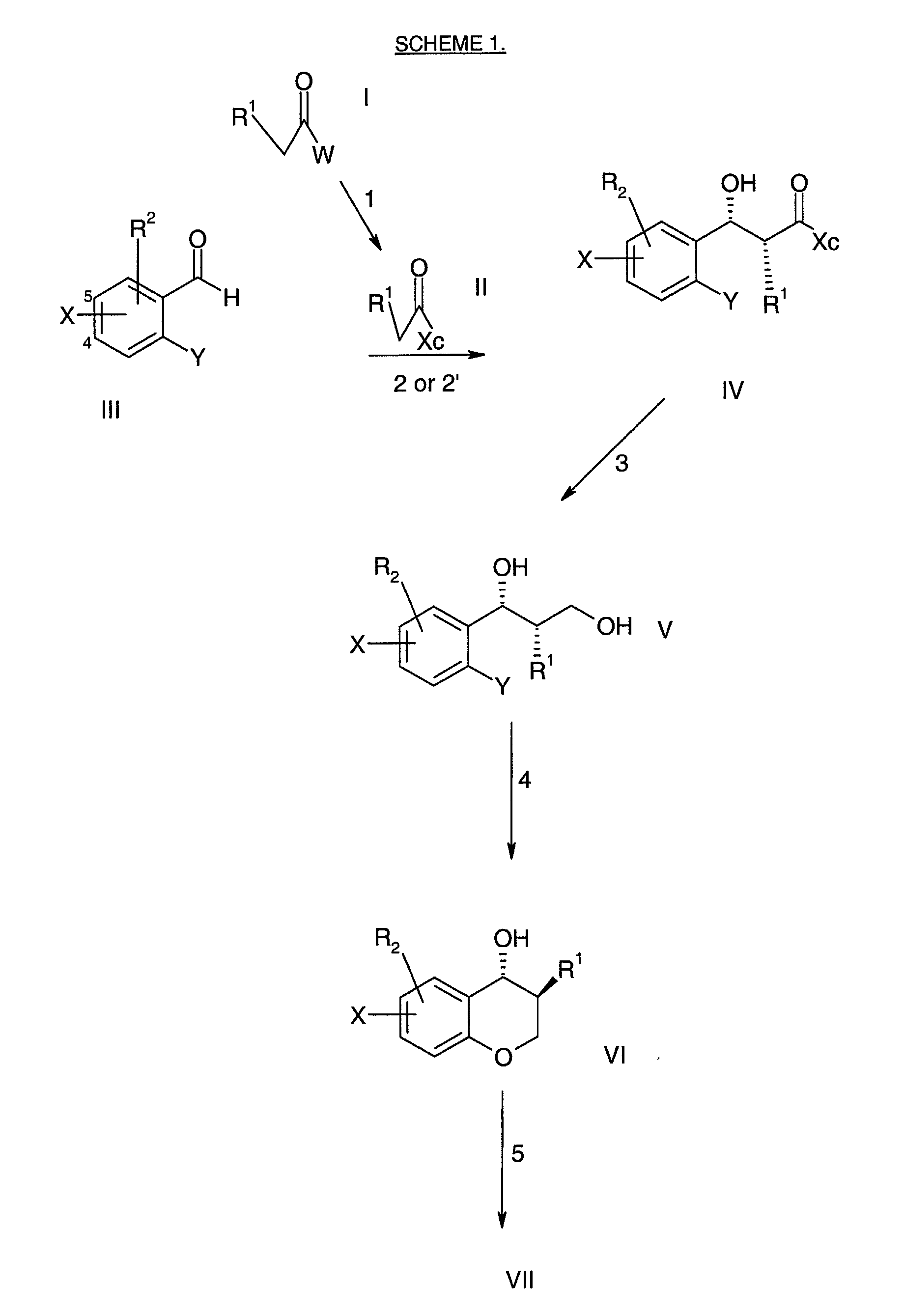

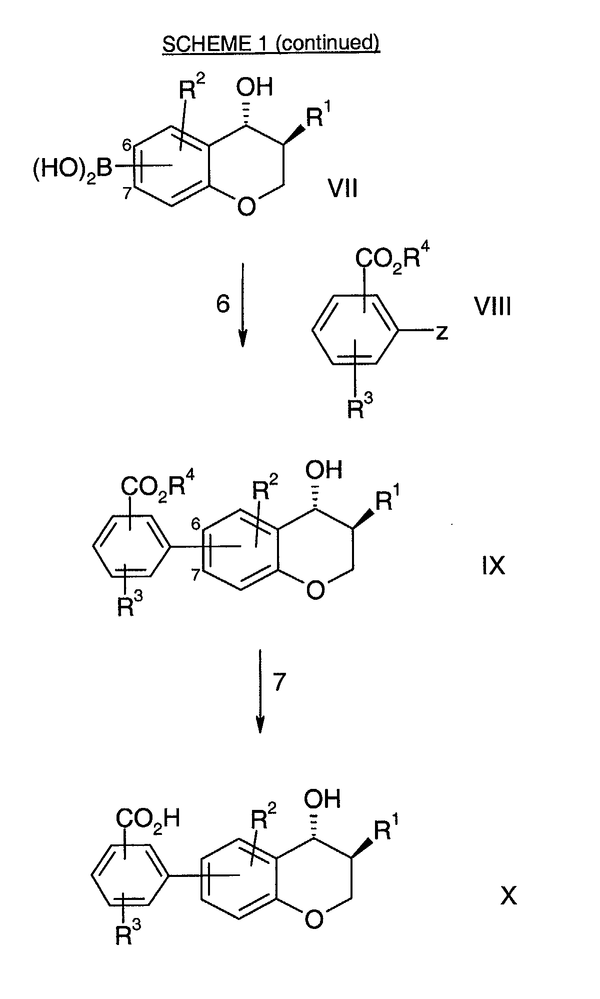

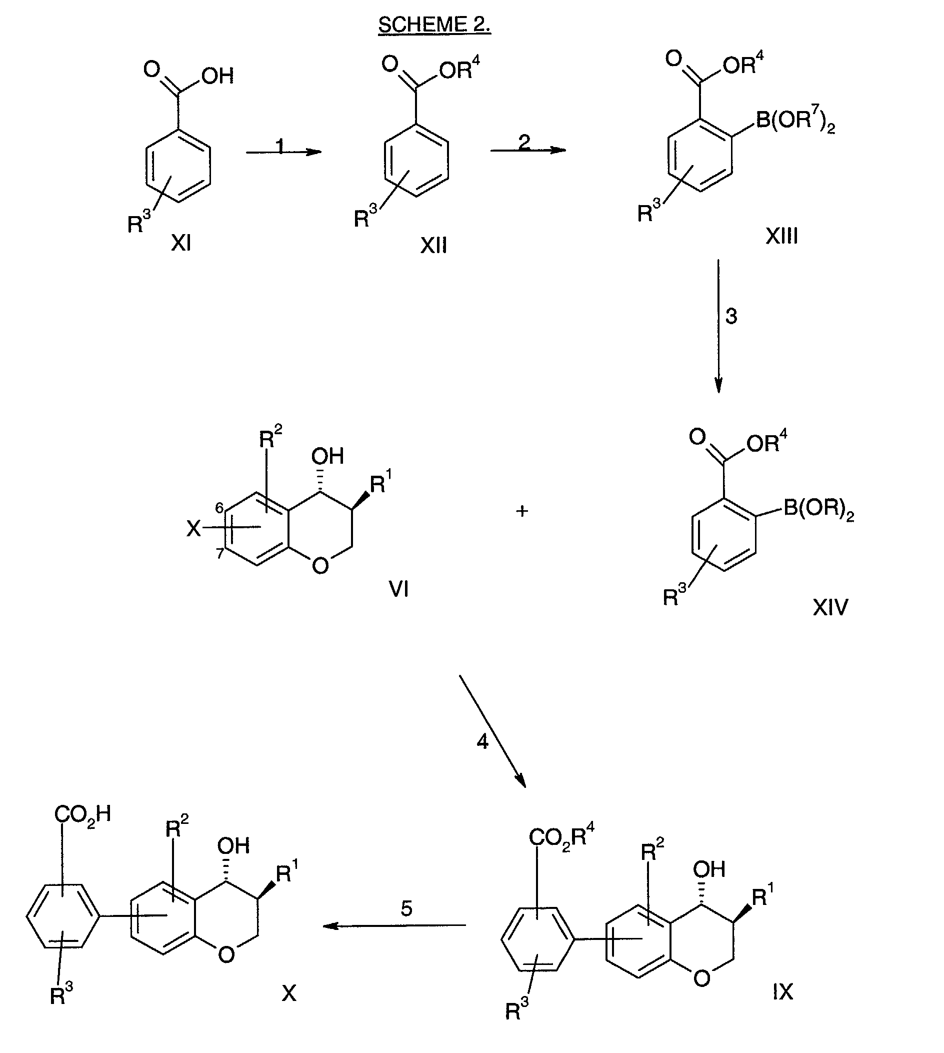

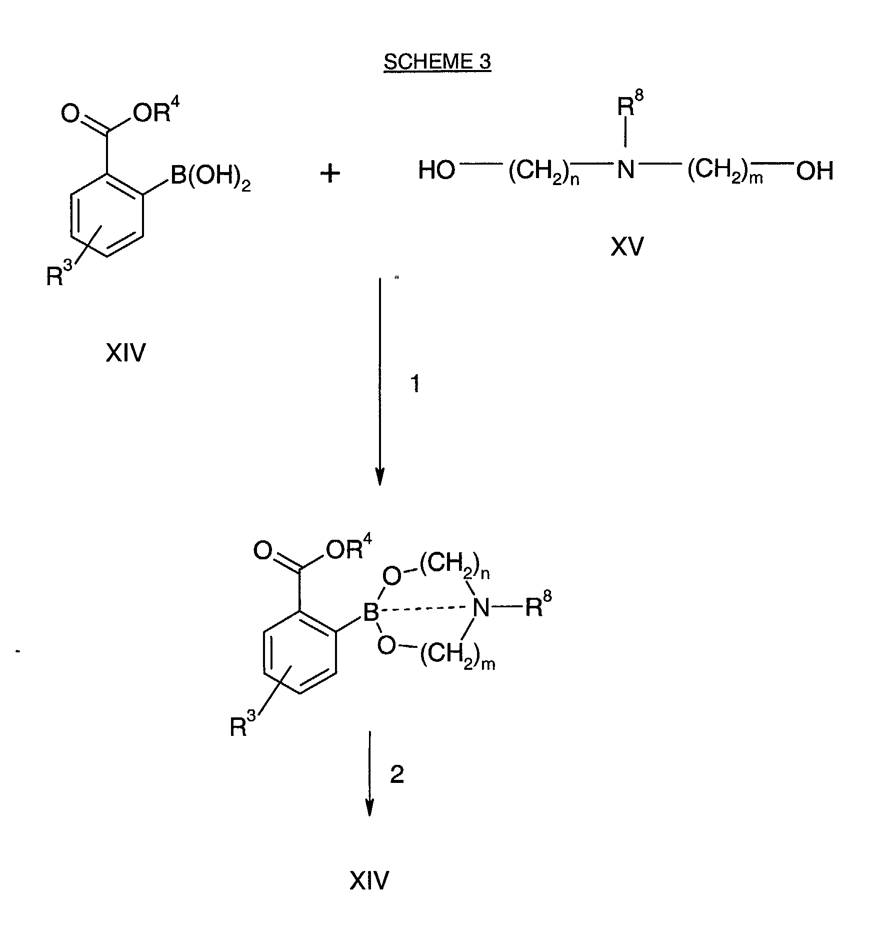

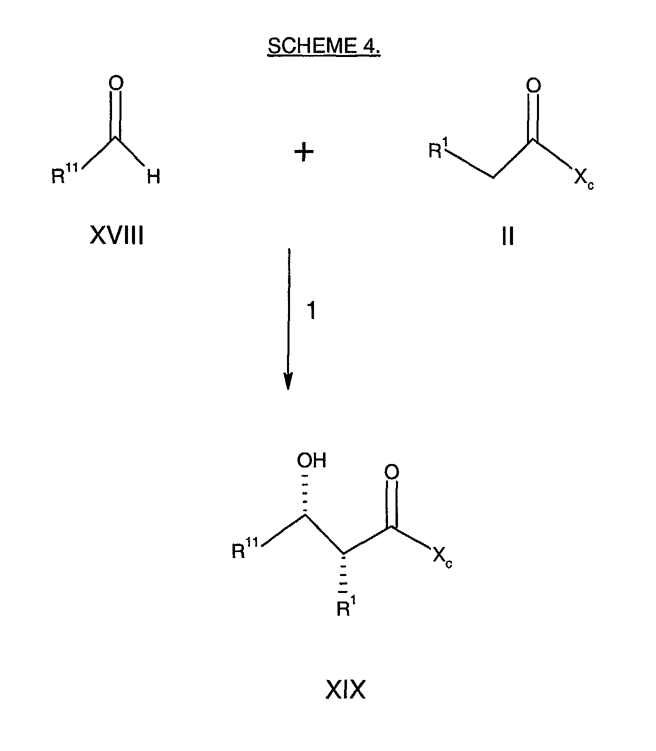

Overall, the synthetic sequence in Scheme 1 involves attaching chiral auxiliary X_{c} to R¹-containing compound **I** (step 1), asymmetric aldol condensation with aldehyde **III** (step 2 or 2'), reductive removal of the chiral auxiliary from aldol **IV** (step 3), base-mediated cyclization of diol **V** (step 4), lithiation and boration of halobenzopyranol **VI** (step 5), coupling boronic acid **VII** with aryl halide or sulfonate **VIII** (step 6), and hydrolysis of ester **IX** (step 7).

Step 1 of Scheme 1 illustrates the conversion of chiral auxiliary HX_{c} to the corresponding anion by treatment with a suitably strong base, such as an alkyllithium base, preferably butyllithium, in an aprotic solvent, such as an ethereal solvent, preferably tetrahydrofuran (THF), at a temperature of approximately -80 to 0°C, preferably -78 to -55°C, over a period of about 20 minutes to one hour. Substituent X_{c} is a chiral auxiliary that is suitable to control relative and absolute stereochemistry in asymmetric aldol reactions. Examples of HX_{c} include (*R*)-4-benzyl-2-oxazolidinone, (*S*)-4-benzyl-2-oxazolidinone, (4*R*,5*S*)-4-methyl-5-phenyl-oxazolidin-2-one, and (4*S*,5*R*)-4-methyl-5-phenyl-oxazolidin-2-one. The resulting anion is treated with acylating agent **I**, wherein group W is a halo, preferably chloro, and R¹ is as defined above, in the same solvent at a temperature of approximately -80 to 0°C, preferably about -75°C, over a period of about one hour, and then warmed to approximately -20 to 20°C, preferably about 0°C, before aqueous workup, which is preferably done by treatment with aqueous sodium bicarbonate, to yield acylated chiral auxiliary **II**.

Step 2 of Scheme 1 illustrates an "Evans aldol" reaction that is performed under conditions that are analogous to those described in Evans, D. A.; Bartroli, J.; Shih, T. L., *J. Am. Chem. Soc.* **1981**, *103,* 2127 and Gage, J. R.; Evans, D. A., *Org. Syn.* **1989**, 68, 83, both of which references are incorporated herein by reference. In particular, in step 2 of Scheme 1, the acylated chiral auxiliary **II** is treated with a Lewis acid, a base, and substituted benzaldehyde **III** to yield alcohol **IV** with a high degree of stereoselectivity. Benzaldehyde **III** is substituted with ortho substituent Y which serves as a leaving group during cyclization step 4, group X (or X' for Scheme 2, in particular coupling step 4 of Scheme 2) which is substituted by the aryl sidechain during coupling step 6, and substituent R² which is as defined above. Substituent X (or X' for Scheme 2) is attached at position 4 or 5 of the phenyl moiety of benzaldehyde **III**. The leaving group Y is typically a halo or nitro group and X is a halide (and, for Scheme 2, X' is a halide or C₁-C₄ perfluoroalkylsulfonate). To prepare aldol product **IV,** acylated chiral auxiliary **II** is treated with a boron halide or sulfonate, such as a dialkylboron sulfonate, preferably dibutylboron triflate, in an aprotic solvent, such as dichloromethane, 1,2-dichloroethane, toluene, or diethyl ether, preferably dichloromethane, at a temperature of about -78 to 40°C, preferably -5°C, over a period of about 20 minutes, followed by treatment with a tertiary amine base, such as triethylamine or diisopropylethylamine, preferably triethylamine, at a temperature of about -78 to 40°C, preferably -5 to 5°C, over a period of about one hour. This mixture is treated with substituted benzaldehyde **III** at a temperature of about -100 to 0°C, preferably about -70°C, over a period of about 30 minutes. This mixture is allowed to warm to a temperature of about -20 to 25°C, preferably about -10°C, over a period of about one hour, and then treated with a protic oxidative quench, preferably by the successive addition of a pH 7 buffer solution, methanol, and aqueous hydrogen peroxide, at a temperature of less than about 15°C, to yield alcohol **IV**.

Step 2' of Scheme 1 illustrates an alternative, and preferable, method of providing alcohol **IV** using a titanium-containing Lewis acid. In step 2' of Scheme 1, acylated chiral auxiliary **II** is treated with a titanium(IV) halide, preferably titanium tetrachloride, in an aprotic solvent such as dichloromethane, 1,2-dichloroethane, or toluene, preferably dichloromethane, at a temperature of about -80 to 0°C, preferably -80 to -70°C, over a period of about 30 minutes with additional stirring for about 30 minutes, followed by treatment with a tertiary amine or tertiary diamine base, such as triethylamine or *N,N,N',N'*-tetramethylethylenediamine, preferably *N*,*N*,*N*',*N*'-tetramethylethylenediamine, at a temperature of about -80 to 0°C, preferably -80 to -65°C, over a period of about 30 minutes. This is optionally, and preferably, followed by treatment with a donor ligand, such as 1-methyl-2-pyrrolidinone, dimethylformamide, 1,3-dimethyl-3,4,5,6-tetrahydro-2(1 H)-pyrimidinone, triethylphosphate, or 2,2'-dipyridyl, preferably 1-methyl-2-pyrrolidinone, at a temperature of about -80 to 0°C, preferably -80 to -65°C, followed by stirring for a period of about 30 minutes. This mixture is treated with substituted benzaldehyde **III** at a temperature of about -100 to 0°C, preferably -80 to -65°C, over a period of about 30 minutes, and allowed to warm to a temperature of -30 to 30°C, preferably 0 to 25°C, over a period of about one to 24 hours, preferably about 4 hours. This mixture is treated with a protic quench, preferably aqueous ammonium chloride, at a temperature of -30 to 30°C, preferably 0 to 25°C, to yield alcohol **IV**. Where treatment with a donor ligand is done, the alcohol **IV** is, in some cases, provided as a crystalline solvate with the donor ligand. Stirring the quenched reaction mixture with a solid support such as Celite® for a period of about 12 hours at a temperature of about 20 °C improves the filtration of the reaction mixture for removal of titanium byproducts.

The titanium aldol conditions of step 2' of Scheme 1 are preferable and operationally more simple than the boron aldol conditions of step 2 of Scheme 1 in that they avoid the pyrophoric reagent tributylborane, the corrosive reagent triflic acid, and their exothermic combination in the preparation of the Lewis acid dibutylboron triflate.

Further, in contrast to titanium aldol reactions described in the literature, such as in Evans, D. A.; Rieger, D. L.; Bilodeau, M. T.; Urpi, F., *J. Am. Chem. Soc.* **1991**, *113*, 1047, the titanium aldol conditions of step 2' of Scheme 1 provide high selectivity with less than two equivalents of the aldehyde **III**. Preferably, about one equivalent of aldehyde **III** is used in this step. The phrase "about one equivalent" as used herein in reference to aldehyde **III** or a compound of the formula R¹¹C(O)H (as recited in the claims) means less than 1.5 equivalents of said compound. In the foregoing article by Evans *et. al.*, it is reported that two equivalents of aldehyde would be required for a titanium aldol reaction analogous to step 2' of Scheme 1.

In addition to having utility in the preparation of the compound of formula **X**, the titanium aldol conditions of step 2' of Scheme 1 are useful in the preparation of HIV protease inhibitor compounds that are described in United Kingdom patent application number 2,270,914 (published March 30, 1994) and in B.D. Dorsey et. al., Tetrahedron Letters, **1993,** 34(12), 1851. The reaction of Scheme 4 can be used to prepare the HIV protease inhibitor compounds that are described in said United Kingdom patent application, wherein R¹¹ is C₁-C₉ alkyl or C₂-C₉ alkenyl, preferably 3-cyclohexylpropenyl. Specifically, Scheme 4 illustrates the application of titanium aldol reaction to aldehyde **XVIII** in which R¹¹ is C₁-C₉ alkyl, C₂-C₉ alkenyl, or phenyl substituted by Y in the 2 position, X in the 4 or 5 position, and R² in one of the remaining positions of the phenyl moiety, wherein Y, X and R² are as defined above. The reaction conditions for Scheme 4 are the same as those described above for step 2' of Scheme 1. Aldehyde **XVIII** encompasses aldehyde **III** from Scheme 1, and alcohol **XIX** encompasses alcohol **IV** from Scheme 1.

Table 1 below illustrates how the product of step 2' of Scheme 1 (or the product of Scheme 4) can vary depending on the reaction conditions that are used, and, in particular, how the diastereoselectivity increases by increasing the amount TMEDA from 1.2 to 3 equivalents and by the addition of 2 equivalents of NMP. Referring to Table 1, 1.0 equivalent of aldehyde RCHO was used for each reaction, x and y represent equivalents of base and NMP, respectively, NMP means 1-methyl-2-pyrrolidinone, TMEDA means *N,N,N',N'*-tetramethylethylenediamine, NEtiPr₂ means diisopropylethylamine, and the ratio of diastereomers was determined by HPLC. The aldol isomers were identified by separation and conversion to known carboxylic acid isomers by hydrolysis with LiOH/H₂O₂ according to procedures analogous to those described in Van Draanen, N. A.; Arseniyadis, S.; Crimmins, M. T.; Heathcock, C. H., *J. Org. Chem.* **1991,** *56,* 2499 and Gage, J. R.; Evans, D. A., *Org. Syn.* **1989,** *68,* 83. The desired isomer is indicated in bold.

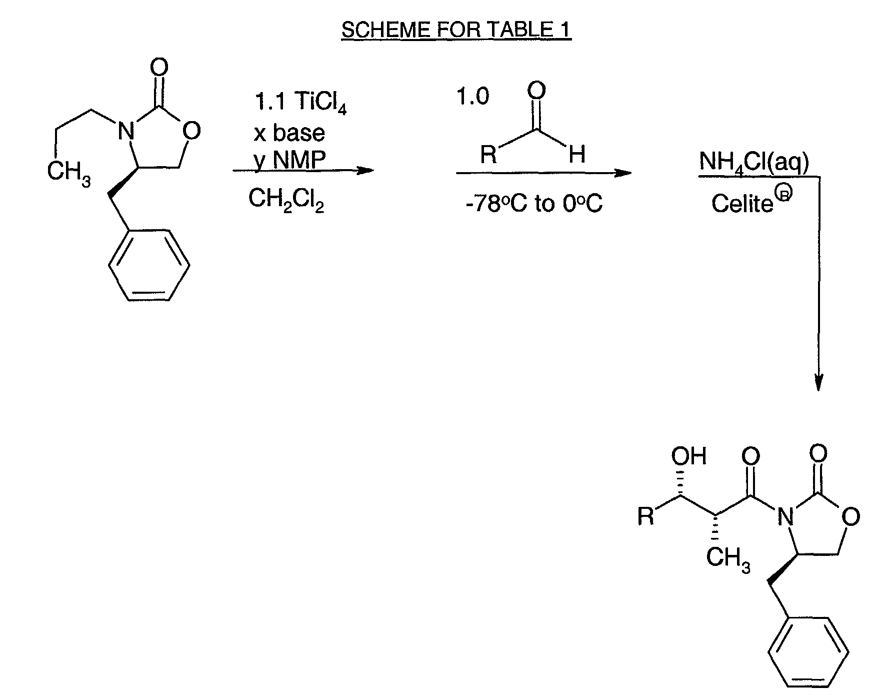

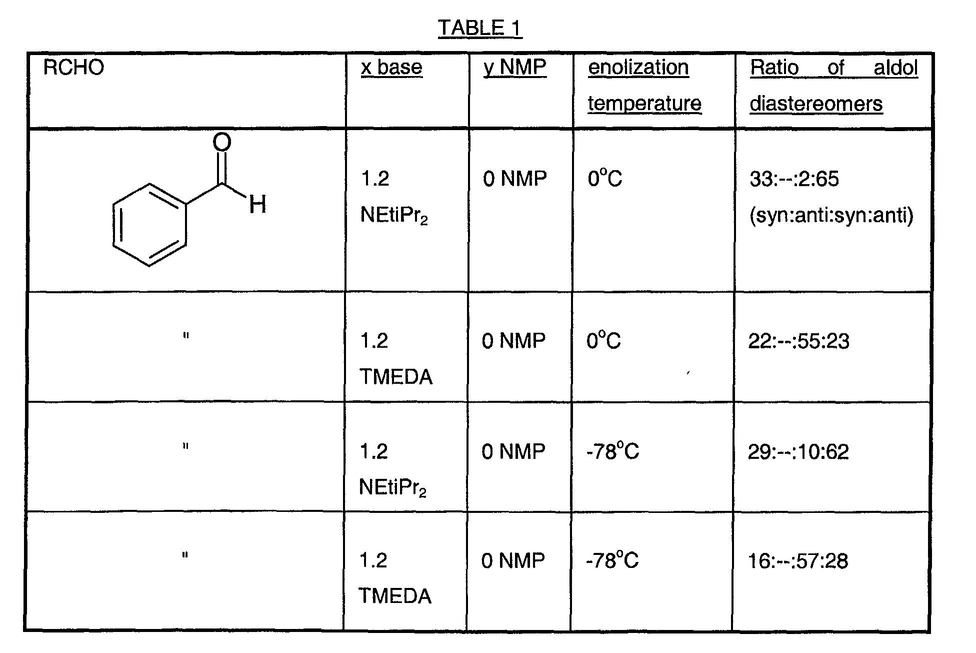

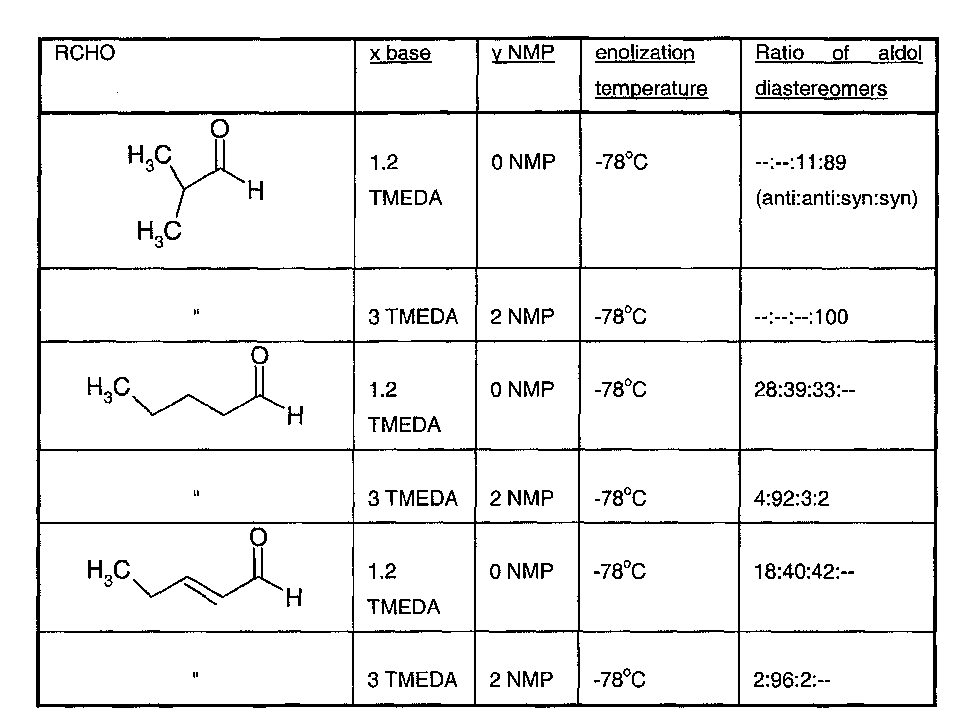

Step 3 of Scheme 1 illustrates the removal of chiral auxiliary X_{c}, which is optionally recovered for reuse in step 1, and the reduction of compound **IV** (acid level) to the desired alcohol **V** according to a procedure analogous to the procedure described in Penning, T. D.; Djuric, S. W.; Haack, R. A.; Kalish, V. J.; Miyashiro, J. M.; Rowell, B. W.; Yu, S. S., *Syn. Commun.* **1990,** *20*, 307, which is incorporated herein by reference. In this process, alcohol **IV** is treated with a hydride reducing agent, such as lithium borohydride, lithium aluminum hydride, sodium borohydride, or calcium borohydride, preferably lithium borohydride, in an ethereal solvent such as THF, diisopropyl ether, or methyl tert-butyl ether, preferably THF, typically containing a protic solvent, such as water, ethanol, or isopropanol, at a temperature of about -78°C to reflux temperature, preferably 0°C to room temperature (20-25°C). After a period of one to 24 hours, typically 12 hours, the reaction is quenched with water with the optional subsequent addition of hydrogen peroxide. Chiral auxiliary HX_{c} can be recovered for reuse in step 1 by selective precipitation, or by extraction of HX_{c} into aqueous acid, preferably hydrochloric acid, from a solution of diol **V** in an organic solvent such as diisopropyl ethyl or a mixture of ethyl acetate and hexane, followed by neutralization of the aqueous acidic extracts with base, and extraction of HX_{c} into an organic solvent.

Step 4 of Scheme 1 illustrates an intramolecular aromatic substitution whereby the primary hydroxyl in diol **V** displaces ortho leaving group Y to generate the benzopyranol ring system of **VI**. In particular, diol **V**, in which leaving group Y is a halo or nitro group, preferably a fluoro group, is treated with a base, such as potassium *tert*-butoxide, sodium bis(trimethylsilyl)amide, potassium bis(trimethylsilyl)amide, cesium carbonate, or sodium hydride, preferably potassium *tert*-butoxide, in an aprotic solvent such as THF, dimethyl sulfoxide, or 1-methyl-2-pyrrolidinone, preferably THF, optionally in the presence of added copper salts, at a temperature of between room temperature and 130°C, preferably about 70°C, for a period of one to 24 hours, typically about four hours, giving benzopyranol **VI**. In benzopyranol **VI**, the substituent X (or X' for Scheme 2) is attached at position 6 or 7 of the benzopyran ring.

Step 5 of Scheme 1 illustrates the conversion of substituent X in benzopyranol **VI** to lithium and then to a boronic acid group. For lithiation, benzopyranol **VI** is preferably treated first with methyl lithium to form the lithium alkoxide followed by butyl lithium to form the aryl lithium. In this process, benzopyranol **VI**, in which X is a halide, preferably bromide or iodide, is treated with two equivalents of alkyllithium, preferably first with one equivalent of methyllithium followed by one equivalent of butyl lithium, in an ethereal solvent, preferably THF, at a temperature of -78 to 0°C, preferably -70 to -65°C, for a period of about one hour, followed by treatment with a borating agent, such as borane-tetrahydrofuran complex, triisopropyl borate, or trimethyl borate, preferably borane-THF complex, at a temperature of -78 to 0°C, preferably -70 to -65°C, over a period of about 30 minutes, followed by quenching with water or optionally aqueous acid at a temperature of about -65°C to room temperature, preferably at about 0°C, giving boronic acid **VII** in which the boronic acid moiety is attached at position 6 or 7 of the benzopyran ring.

Step 6 of Scheme 1 illustrates a Suzuki coupling between boronic acid **VII** and compound **VIII** to form the biaryl bond of compound **IX.** In compound **VIII**, Z is a halide or sulfonate, preferably bromide, iodide, or trifluoromethanesulfonate, R⁴ is C₁-C₆ alkyl and R³ is as defined above. This procedure is analogous to the procedure described in Miyaura, N.; Suzuki, A., *Chem. Rev.* **1995**, *95,* 2457, which is incorporated herein by reference. This procedure is preferable to the coupling of zinc or tin species due to the difficulty of preparing organozincs on a large scale and the toxicity of organotin compounds. In this process, a mixture of boronic acid **VII**, arene **VIII**, a palladium catalyst, such as tetrakis(triphenylphosphine)palladium(0), dichlorobis(triphenylphosphine)palladium(II), palladium(II) acetate, allylpalladium chloride dimer, tris(dibenzylideneacetone)dipalladium(0), or 10% palladium on carbon, preferably 10% palladium on carbon, and a base or fluoride salt, such as sodium carbonate, triethylamine, sodium bicarbonate, cesium carbonate, tripotassium phosphate, potassium fluoride, cesium fluoride, or tetrabutylammonium fluoride, preferably potassium fluoride, in a solvent such as ethanol, dimethoxyethane, or toluene, optionally containing water, preferably ethanol, are stirred at a temperature of between room temperature and 130°C, preferably reflux temperature, for a period of about one to about 24 hours, preferably about three hours, giving biaryl **IX** in which the benzyl ester moiety is attached at position 6 or 7 of the benzopyran ring.

Step 7 of Scheme 1 illustrates the treatment of ester **IX** with aqueous hydroxide base, such as aqueous sodium hydroxide, in an alcoholic solvent, such as isopropyl alcohol, at a temperature of between 40°C and reflux temperature, preferably reflux temperature, for a period of about one to about 24 hours, preferably about six hours. The reaction mixture is cooled to room temperature and partitioned between aqueous base and an organic solvent, such as a mixture of hexane and isopropyl ether. The aqueous solution is acidified, and the final compound **X** is extracted into an organic solvent such as ethyl acetate. This method of extracting the compound **X** with organic solvents removes neutral impurities which is particularly advantageous in the last step of this synthesis.

To facilitate the handling of carboxylic acid **X**, this compound can be treated with a secondary amine of the formula NHR⁵R⁶, wherein R⁵ and R⁶ are as defined above, in a solvent such as toluene, to form an ammonium carboxylate of the formula 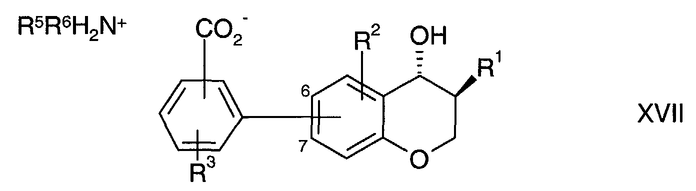 wherein R¹, R², R³, R⁵ and R⁶ are as defined above. Ammonium carboxylate **XVII** can be treated with an aqueous acid such a hydrochloric acid or sulphuric acid, preferably hydrochloric acid, in a solvent such as ethyl acetate, toluene, or methylene chloride, preferably ethyl acetate, at a temperature ranging from O°C to room temperature for a period of 30 minutes to 3 hours, preferably 1 hour, to provide carboxylic acid **X**.

Scheme 2 illustrates an alternative to the coupling sequence of steps 5 and 6 of Scheme 1. The process of Scheme 2 is preferred. Step 1 of Scheme 2 is an esterification of carboxylic acid **XI** with alcohol R⁴OH, in which R³ and R⁴ are as defined above, to generate ester **XII**. In this process, carboxylic acid **XI** is treated with alcohol R⁴OH, preferably a primary or secondary alcohol such as 2,2-dimethyl-propyl alcohol, and an acid such as sulfuric acid, hydrochloric acid, methanesulfonic acid, toluenesulfonic acid, or camphor sulfonic acid, preferably sulfuric acid, in a solvent such as toluene, dichloromethane, or dichloroethane, preferably toluene, at a temperature of 0°C to reflux temperature, preferably reflux temperature, for a period of one to 24 hours, typically 4 hours, to provide ester **XII**.

Step 2 of Scheme 2 illustrates the treatment of ester **XII** with a base and the resulting ortho metallated species is trapped with a trialkylborate to give boronate ester **XIII**. In step 3 of Scheme 2, the boronate ester **XIII** is hydrolyzed to the corresponding boronic acid **XIV** which is performed by methods known to those skilled in the art. In steps 2 and 3 of Scheme 2, ester **XII** is treated with a metal amide base such as lithium diisopropylamide, lithium diethylamide, lithium 2,2,6,6-tetramethylpiperidine, or bis(2,2,6,6-tetramethylpiperidino)magnesium, preferably lithium diisopropylamide, in the presence of a tri-(C₁-C₄ alkyl)borate, such as triisopropylborate, triethylborate, or trimethylborate, preferably triisopropylborate, in an ethereal solvent, such as THF, diisopropyl ether, dioxane, or methyl tert-butyl ether, preferably THF, over a temperature range of about -78°C to room temperature (20-25°C), preferably about 0°C. After a period of 10 minutes to 5 hours, typically 1 hour, the reaction is quenched with aqueous acid to provide boronic acid **XIV**.

To facilite the handling of boronic acid **XIV** before proceeding to step 4 of Scheme 2, the boronic acid **XIV** can be reacted with an aminodiol as illustrated in Scheme 3. In Scheme 3, boronic acid **XIV** is reacted with aminodiol **XV**, wherein R⁸, m and n are as defined above, in a solvent such as isopropanol, ethanol, methanol, hexanes, toluene, or a combination of the foregoing solvents, preferably isopropanol, at a temperature within the range of 0°C to reflux temperature, preferably room temperature, for a period of 15 minutes to 10 hours, preferably 10 hours, to provide the amine complex **XVI**. To proceed with step 4 of Scheme 2, amine complex **XV** is hydrolyzed to boronic acid **XIV** according to methods known to those skilled in the art. Such methods include the use of aqueous acid, such as hydrochloric acid.

Step 4 of Scheme 2 illustrates a Suzuki coupling between boronic acid **XIV** and benzopyranol **VI** to form the biaryl bond of **IX.** In this process, a mixture is prepared containing boronic acid **XIV**, benzopyranol **VI**, a palladium catalyst, such as tetrakis(triphenylphosphine)palladium(0), dichlorobis(triphenylphosphine)palladium(II), palladium(II) acetate, allylpalladium chloride dimer, tris(dibenzylideneacetone)dipalladium(0), or 10% palladium on carbon, preferably tetrakis(triphenylphosphine)palladium(0), a base or fluoride salt, such as sodium carbonate, triethylamine, sodium bicarbonate, cesium carbonate, tripotassium phosphate, potassium fluoride, cesium fluoride, sodium hydroxide, barium hydroxide, or tetrabutylammonium fluoride, preferably sodium carbonate, and a solvent such as toluene, ethanol, dimethoxyethane, optionally containing water, preferably toluene containing water. In benzopyranol **VI,** which is prepared according to Scheme 1, X', which is attached at position 6 or 7 of the benzopyran ring, represents a halide or C₁-C₄ perfluoroalkylsulfonate, preferably bromide, iodide, or trifluoromethanesulfonate. The mixture is stirred at a temperature of between room temperature and reflux temperature, preferably reflux temperature, for a period of about 10 minutes to about 6 hours, preferably 1 hour, to provide biaryl **IX**.

Step 5 of Scheme 2 illustrates the hydrolysis of ester **IX** to provide the carboxylic acid **X** as described above for step 7 of Scheme 1.

The salts of compound of formula I may be prepared in a conventional manner by reaction with a base such as an alkali metal hydroxide, e.g., sodium hydroxide, or alkaline earth metal hydroxide, e.g., magnesium hydroxide.

### EXAMPLES

The invention will be better understood by making reference to the following specific examples.

*Mouse line derivation.* Genetically modified apolipoprotein E-deficient (ApoE^{-/-}), low density lipoprotein (LDL) receptor-deficient (LDLr^{-/-}), and monocyte chemoattractant protein-1-deficient (MCP-1^{-/-}) mice used in the present study have been previously described and characterized [See Rutledge, B.J. et. al., *J. Immunol.*, 1995;155:4838-4843; and Nakashima Y, et. al., *Arterioscler. Thromb.*, 1994;14(1):133-140]. To obtain mice homozygous for both the apoE and MCP-1 (ScyA2) null alleles, male ApoE^{-/-} mice on a C57B/6 background (C57E⁻ ^{/-}) were mated with heterozygous female MCP-1^{-/+} mice on a C57B/6 background. The resulting ApoE^{-/-} x MCP-1^{-/+} progeny were identified by Southern blot analysis and inbred to produce ApoE^{-/-} x MCP-1^{-/-} (C57E^{-/-} x MCP-1 ^{-/-}) and control ApoE ^{-/-} x MCP-1^{+/+} (C57E-/-) mice. ApoE^{-/-} mice on the mixed genetic background 1290la x C57B/6 (129E^{-/-}) were bred in-house and derived from brother and sister matings. All mice were weaned at 21 days, maintained on a 12 hour light/dark cycle and fed either a chow or a Western-type diet [See, Bourassa, P.K., et. al., *Proc Natl Acad. Sci. USA,* 1996;93:10022-10027].

*Administration of Compound.* Compound of formula I, preferably (3S, 4R)-7-(2-carboxy-5-trifluoromethylphenyl)-4-hydroxy-3-benzyl-3,4-dihydro-2H-1-benzopyran, was evaluated for its effects on lesion development in age-matched groups of mice (15 to 30 weeks of age at time of sacrifice) using a 35 day treatment protocol. Subgroups of mice were treated with (3S, 4R)-7-(2-carboxy-5-trifluoromethylphenyl)-4-hydroxy-3-benzyl-3,4-dihydro-2H-1-benzopyran for shorter times (7-14 days) for evaluation of acute changes in tissue gene expression as noted in the table and figure legends. In all study groups, the compound was administered by oral gavage once daily in a vehicle comprised of 0.6% Tween 80 + 0.25% methylcellulose in PBS. Mice received either vehicle alone, 30mg/kg/day, or 100mg/kg/day of compound of formula I.

*CD11b FACs Analysis.* After treatment period, mice were anesthetized with ketamine:xylazine:PBS (1:1:2) and a whole blood sample was collected from the abdominal aorta into vacutainers containing 7.5% EDTA. Determination of blood monocyte CD11b expression was performed as described previously [See Showell, H.J., et. al., *Journal of Pharmacoloty and Experimental Therapeutics*, 1995;273:176-184]. Cells were pelleted by gentle centrifugation (1200 rpm, 2 minutes) and incubated with 50 µg/ml of fluorescein-conjugated rat anti-mouse CD11b IgG (Pharmingen) at room temperature for 30 minutes. To control for nonspecific binding, duplicate samples were incubated with 50 µg/ml of fluorescein-conjugated rat IgG. After a 30 minute incubation, cells were washed with one ml HiFAZ buffer, pelleted, and red blood cells lysed with a lysing buffer (Becton Dickinson, Mountain View, CA) for 10 minutes at room temperature. The cells were then washed twice, resuspended in HiFAZ buffer, and the degree of fluorescence was determined on FACs flow cytometer. Data were collected in the list mode and macrophage gates were defined by the forward scatter versus side scatter dot blots.

*Plasma lipoproteins and lipids.* Separation of plasma lipoproteins using fast protein liquid chromatography (FPLC, Pharmacia LKB Biotechnology, Inc., Piscataway, NJ) was performed as previously described [See Bourassa, P.K., et. al., *Proc Natl. Acad. Sci. USA,* 1996;93:10022-10027]. Total plasma cholesterol and triglycerides were measured using colorimetric methods with commercially available Cholesterol/HP (Boehringer-Mannheim) and Triglycerides G (Wako Chemical) kits.

*Aortic Tree Analysis.* In some experiments, mice were perfusion-fixed with 4% paraformaldehyde and the entire aortic tree was removed including the brachiocephalic region and the carotid, subclavian and femoral branches. The tissue was cleaned of adventitia and laid out on a piece of polystyrene and a digital image was obtained using a CCD camera attached to a copy stand. The carotid and subclavian branches were removed and the brachiocephalic region was isolated for further processing. The percent of the aortic surface covered by lesions in the remaining portion of the aortic tree was determined using an En face preparation as previously described [See Bourassa, P.K., et. al., *Proc Natl Acad. Sci. USA*, 1996;93:10022-10027]. Each aorta was evaluated for lesion area by direct image capture and display on a Trinitron monitor. The lesion area was determined in unstained tissue using ImagePro 3.1 image analysis (Image Processing Solutions, Woburn, MA). Areas of atherosclerotic plaques in aortas cleaned of adventitia appear as yellowish-white opaque areas compared to translucent non-lesioned areas. This area was quantified by manually setting thresholds for shades of black (background), gray (normal tissue), and white (lesion area).

*Lesion Analysis.* To determine cross-sectional lesion area, hearts were perfusion-fixed in 4% paraformaldehyde, infiltrated with 30% gum sucrose for 24 hours at 4°C, and embedded in OCT compound and sectioned 10µm, -18°C, as previously described [See Borgeat P., et.al., *J. Biol. Chem.*, 1979;254:2643-2646] and the sections were stained with Oil red O (Polyscientific, Bay Shore NY) and counterstained with Gill III hematoxylin (Sigma, St. Louis MO). Each section of the aortic valve was evaluated for Oil red O staining area by capturing images directly from a RGB camera attached to a Leitz Laborlux S (Leica) light microscope for display on a Trinitron RGB monitor. Image analysis was performed using ImagePro 3.1' software as described previously [See Bourassa, P.K., et. al., *Proc Natl. Acad. Sci. USA*, 1996;93:10022-10027]. Results are expressed as the average lesion size per section or as the percent of the total cross sectional vessel wall stained with Oil red O. For each animal, the average lesion area of 12-16 sections was determined and data are expressed as lesion size or mean percent lesion area ±S.D.

*Immunohistochemical Staining of Macrophages.* Serial paraformaldehyde fixed, OCT embedded sections of aortic valves were immunostained for macrophages using rat monoclonal antibodies (IgG_{2b}) against MOMA-2 (BioSource) and CD11b (Pharmingen). Endogenous biotin and peroxidase activity were blocked by incubating each section with an avidin/biotin solution (Vector Laboratories), and 0.3% H₂O₂ in 1% bovine serum, respectively. Rat, anti-mouse MOMA-2 was applied at a concentration of 25 µg/ml, and rat-anti-mouse CD11b was applied at a concentration of (5 µg/ml). Incubation with primary antibodies was followed by a biotinylated donkey anti-rat secondary antibody IgG (1.4 µg/ml, Jackson ImmunoResearch), and incubation with horseradish peroxidase-conjugated streptavidin (1:500). An non-specific rat IgG2b antibody (Pharmingen) was used as a negative control. Antibody binding was visualized with DAB (Vector Laboratories), and all sections were counterstained with Gill III hematoxylin. Results are expressed as the percent of the total cross-sectional vessel wall area (normal + diseased area/ section, excluding the lumen) stained with DAB.

*Peritoneal macrophage chemotaxis assay.* Upon the termination of the study, a subset of mice from each group were injected intraperitoneally with one ml of sterile 6% casein, and after four days peritoneal exudate cells were harvested by washing the peritoneal cavity with Hanks Balanced Salt Solutions HBSS (Life Technologies), supplemented with 1% Fetal Bovine Serum (FBS). Peritoneal cells then were washed three times in RPMI-1640 supplemented with 0.1% bovine serum albumin BSA. For chemotaxis, cells were suspended at a concentration of 2 x10⁶ cell/ml in chemotaxis buffer (RPMI-1640 supplemented with 0.1% BSA and 20mM HEPES (pH 7.4)). The cell suspension was loaded in the upper chamber of a 48-well microtaxis chamber. Murine MCP-1 (10 nM or LTB₄ (10 nM) was added to the lower chamber, which was separated from the upper chamber by a 5-µm polycarbonate membrane. The peritoneal macrophages attached to the underside of the membrane were fixed and stained using the Diff-Quick stain set (Dade Behring Inc, Switzerland). The results were expressed as the mean number of cells that migrated in four high power fields (20 times) in three replicate samples.

***Statistics.*** Statistical significance was determined by unpaired Student's t test using the StatView statistical program (Abacus Concepts, Inc., Berkeley, CA). Results are reported as mean ± standard deviation.

The activities of the compound of formula I, specifically (3S, 4R)-7-(2-carboxy-5-trifluoromethylphenyl)-4-hydroxy-3-benzyl-3,4-dihydro-2H-1-benzopyran, against atherosclerosis are tabulated in the following Tables 2-4:

**TABLE 2.**

| Effect of LTB₄ antagonism on lesions in atherosclerotic mice. | | | | | | | | | |
|---|---|---|---|---|---|---|---|---|---|
| Strain | N | Sex | Age (wks) | Body wt(gm) | Dose (mg/kg) | TPC (mg/dl) | TG (mg/dl) | Sinus (% lesion) | Valve (%lesion) |
| 129E^{-/-} | 10 | F | 15 | 25±1 | 0 | 739±59 | 72±13 | 22.5±4 | 24.9±3 |
| | 10 | | | 26±2 | 30 | 584±58 | 83±40 | 19.6±2 | 21.6±2 |
| | 5 | | | 25±2 | 50 | 675±181 | 59±17 | 14.7±2* | 16.3±2* |
| | 10 | | | 25±1 | 100 | 858±166 | 36±13 | 7.9±1** | 8.0±2** |
| | 10 | F | 20 | 27±1 | 0 | 795±57 | 120±15 | 34.6±2. | 41.0±3 |
| | 10 | | | 29±1 | 30 | 780±51 | 138±21 | 24.4±3 | 25.9±3* |
| | 5 | F | 24 | 30±1 | 0 | 513±42 | 59±12 | 43.5±3 | 49.5±3 |
| | 5 | | | 31±1 | 100 | 579±37 | 102±9 | 30.0±1 | 34.0±1 |
| C57E^{-/-} | 8 | M | 16 | 29±1 | 0 | 807±83 | 189±73 | 22.9±4 | 24.7±4 |
| | 8 | | | 29±1 | 100 | 801±71 | 169±57 | 10.4±3* | 7.8±2* |
| | 12 | F | 22 | 30± | 0 | 494±51 | 66±5 | 50.3±3 | 56.9±2 |
| | 9 | | | 30± | 100 | 640±41 | 116±6 | 38.8±3 | 39±2 |
| LDLr^{-/-} | 10 | M | 18 | 34±2 | 0 | 1342±315 | 778±210 | 14.8±2 | 15.9±3 |
| | 9 | | | 31±2 | 30 | 798±300 | 532±205 | 5.6±2* | 6.5±3* |

| | | | | | | | | | |
|---|---|---|---|---|---|---|---|---|---|
| * p<. 05 significant difference from Vehicle treated group determined. | | | | | | | | | |

Referring to Table 2, Apolipoprotein E-deficient mice on a mixed 129Io/B6 (129E^{-/-}) or a C57BI/6 background (C57E^{-/-}), mice homozygous low density lipoprotein receptor deficient mice LDLR^{-/-} mice were treated for 35 days orally with (3S, 4R)-7-(2-carboxy-5-trifluoromethylphenyl)-4-hydroxy-3-benzyl-2H-1-benzopyran) at doses indicated. LDLR^{-/-} was fed a Western diet; all other strains were maintained on chow diet. The mean percent lesion area in 10 µ sections of the aortic sinus and valve region was calculated following oil red O staining, Values are reported as means ±SD. The term "ND" is defined as Not Determined. The term "TPC" is defined as Total Plasma Cholesterol. The term "TG" is defined as Triglycerides. The term "sinus" is defined as a region of the aortic válve; moving up from the base of the heart; which begins at the first appearance of the aortic valve cusps, dividing the lumen into three distinct regions. In the sinus, the aortic wall is bulging and irregular. The term "valve" is defined as a region of the aortic valve which begins when the sinus region ends; the valve region begins when the valve cusps no longer divide the lumen and the aortic wall appears more rounded and distinct. The valve regions end when the valve cusps are no longer apparent and the wall is well rounded.

**Table 3.**

| Effect of LTB₄ antagonism on CD11b in atherosclerotic lesions. | | |
|---|---|---|
| Strain | Lesion size µM | % CD11b |
| 129E^{-/-} | 188175±19110 | 12.6±1.1 |
| Treated | 10650±12133 | 3.5±1.0* |
| LDLr^{-/-} | 59009±12734 | 25.1±2.0 |
| Treated | 19955±7278 | 3.5±2.5* |

| | | |
|---|---|---|
| *Significantly different (P<. 01) from control. | | |

Referring to Table 3, female Apolipoprotein-E- deficient mice (129E^{-/-}) maintained on a chow diet and male LDL receptor (LDLr^{-/-}) deficient mice maintained on a Western-type diet were treated with (3S, 4R)-7-(2-carboxy-5-trifluoromethylphenyl)-4-hydroxy-3-benzyl-2H-1-benzopyran) (30 mg/kg/d) orally for 35 days. Data is reported as mean ± SD (N=8/group) lesion area and the mean percent of the lesion staining positively for CD11b. The term "CD11b" is defined as a protein found internally in a cell which forms MAC-1 when coupled with another cellular protein, CD-18; MAC-1 is a receptor on the monocyte surface involved in adhesion.

**Table 4.**

| Effect of LTB₄ antagonism on atherosclerotic lesion in MCP-1 deficient mice. | | | | | | | | |
|---|---|---|---|---|---|---|---|---|
| N | Sex | Age (wks) | Body wt(gm) | Dose (mg/kg) | TPC (mg/dl) | TG (mg/dl) | Sinus (% lesion) | Valve (%lesion) |
| 8 | M | 15 | 29±1 | 0 | 390±20 | 85±11 | 7.6±2 | 5.8±1 |
| 7 | | | 29±1 | 30 | 345±15 | 82±12 | 13.3±2 | 12.8±2 |
| 7 | | | 29±1 | 100 | 354±20 | 79±15 | 9.8±1 | 9.7±1 |
| 14 | M | 30 | 32±1 | 0 | 505±20 | 45±1 | 44.1±4 | 49.6±5 |
| 8 | | | 31±1 | 30 | 565±22 | ND | 41.4±5 | 54.2±7 |
| 7 | | | 26±1 | 100 | 576±21 | 47±2 | 43.1±5 | 50.0±4 |
| No significant difference (P<.05) between vehicle and treated groups. | | | | | | | | |

Referring to Table 4, mice homozygous for the null alleles of Monocyte chemoattractant protein-1 (MCP-1^{-/-}) and apolipoprotein E (ApoE^{-/-}) (MCP-1 ^{-/-} X ApoE^{-/-}) on a C57bl/6 background (C57E^{-/-}) mice were treated for 35 days orally with (3S, 4R)-7-(2-carboxy-5-trifluoromethylphenyl)-4-hydroxy-3-benzyl-2H-1-benzopyran) at doses indicated 0, 30 or 100mg/kg. The mean percent lesion area in 10 µ sections of the aortic sinus and valve region was calculated following oil red O staining, Values are reported as means ±SD. The term "ND" is defined as Not Determined. The term "TPC" is defined as Total Plasma Cholesterol. The term "TG" is defined as Triglycerides. The term "sinus" is defined as a region of the aortic valve; moving up from the base of the heart; which begins at the first appearance of the aortic valve cusps, dividing the lumen into three distinct regions. In the sinus, the aortic wall is bulging and irregular. The term "valve" is defined as a region of the aortic valve which begins when the sinus region ends; the valve region begins when the valve cusps no longer divide the lumen and the aortic wall appears more rounded and distinct. The valve regions end when the valve cusps are no longer apparent and the wall is well rounded.

The compound of formula I used in the methods of the present invention can be synthesized as illustrated by the following preparations, but it is not limited to the details thereof. In the following preparations, the term "room temperature" means a temperature within the range of about 20°C to about 25°C.

### PREPARATION 1

### (3S,4R)-2-(3-Benzyl-4-hydroxy-3,4-dihydro-benzopyran-7-yl)-4-trifluoromethylbenzoic Acid

A mixture of (3*S*,4*R*)-2-(3-benzyl-4-hydroxy-3,4-dihydro-benzopyran-7-yl)-4-trifluoromethyl-benzoic acid ethyl ester (897 g, 1.93 mol) and 10% aqueous sodium hydroxide (980 mL, 2.72 mol) in isopropyl alcohol (9 L) was heated at reflux for 6 hours, cooled to room temperature, and stirred for 12 hours. The reaction mixture was diluted with water (13.5 L), hexanes (9 L), and isopropyl ether (4.5 L). The aqueous layer was separated and extracted with hexanes (9 L) and isopropyl ether (4.5 L), adjusted to pH 2 with 2 N aqueous hydrochloric acid, and extracted with ethyl acetate (8 L and 4 L). The combined ethyl acetate extracts were washed with water (6 L), dried over magnesium sulfate, and concentrated *in vacuo* to a dark amber oil which was diluted with toluene (2 L) and concentrated again to an oil. The oil was dissolved in toluene (4.2 L) at 60°C, and hexanes (8.8 L) were added at a rate to maintain a temperature of greater than 50°C. The tan solids which precipitated upon slowly cooling to room temperature over several hours were filtered and washed with 2:1 hexane/toluene (2 L). These solids were dissolved in toluene (5 L) at 60°C, treated with Darco® G-60, filtered, washed with toluene, and concentrated *in vacuo* to approximately 4.0 L. This mixture was heated to 50-60°C, treated drop-wise with hexanes (8.6 L), cooled, and granulated at 5°C for 1 to 2 hours. The resulting solids were filtered, washed with 2:1 hexanes/toluene (2 L), and the wet cake was stirred with hexanes (4 L) at reflux for 30 minutes. This mixture was cooled to room temperature, granulated for 1 hour, filtered, and the resulting solids were dried under vacuum overnight to provide 450 g (55%) of (3*S*,4*R*)-2-(3-benzyl-4-hydroxy-3,4-dihydro-benzopyran-7-yl)-4-trifluoromethyl-benzoic acid as an off white solid: ¹H NMR (400 MHz, CDCl₃) δ 7.99 (d, J=8.1 Hz, 1H), 7.66 (dd, J=1.1, 8.1 Hz, 1H), 7.63 (s, 1H), 7.15-7.32 (m, 6H), 6.89 (dd, J=1.7, 7.9 Hz, 1H), 6.85 (d, J=1.7 Hz, 1H), 6.1 (bs, 2H), 4.50 (d, J=4.3 Hz, 1H), 4.18 (dd, J=2.7, 11.2 Hz, 1H), 3.94 (dd, J=4.6, 11.0 Hz, 1H), 2.74 (dd, J=6.1, 13.8 Hz, 1H), 2.51 (dd, J=9.4, 13.9 Hz, 1H), 2.22 (m, 1H); IR 3454, 3218 (br), 1699, 1431, 1337, 1299, 1275, 1258, 1191, 1178, 1135, 1123, 700 cm⁻¹; mp 142°C.

The (3S,4R)-2-(3-benzyl-4-hydroxy-3,4-dihydro-benzopyran-7-yl)-4-trifluoromethylbenzoic acid ethyl ester starting material for preparation 1 was prepared as follows:

### A. (R)-4-Benzyl-3-(3-phenyl-propionyl)-oxazolidin-2-one

To a solution of (*R*)-(+)-4-benzyl-2-oxazolidinone (910 g, 5.14 mol) and 500 mg of 2,2'-dipyridyl as an indicator in tetrahydrofuran (9 L) at -78°C was added over 30 minutes a 2.5 M solution of BuLi in hexanes (2.03 L, 5.1 4 mol). The temperature of the reaction mixture was maintained at less than -55°C during the addition. The reaction mixture was cooled to -75°C and hydrocinnamoyl chloride (950 g, 5.63 mol) was added over 5 minutes. The reaction mixture was allowed to warm to 0°C, at which point the reaction mixture was judged to be complete by thin layer chromatography (hexanes/ethyl acetate, 2:1). The reaction was quenched by adding 10% aqueous sodium bicarbonate (3.6 L) and water (3.6 L). The aqueous phase was separated and extracted with ethyl acetate (3 L). The combined organic layers were washed with 5% aqueous sodium carbonate (3.6 L) and saturated aqueous sodium chloride (2 L), dried over magnesium sulfate, and concentrated *in vacuo* to approximately 2 L of a viscous yellow suspension. This slurry was dissolved in ethyl acetate (3 L), concentrated to a solid, and dissolved in ethyl acetate at 50°C. Hexanes (10.7 L) was added, and the mixture was slowly cooled to 10°C resulting in the precipitation of solids which were stirred at 10°C for 30 minutes. The solids were collected by filtration, washed with hexanes, and air-dried at room temperature yielding 1.4 kg (88%) of (R)-4-benzyl-3-(3-phenylpropionyl)-oxazolidin-2-one as pale yellow needles: ¹H NMR (300 MHz, CDCl₃) δ 7.14-7.33 (m, 10H), 4.66 (m, 1H), 4.17 (t, J=3.4 Hz, 2H), 3.26 (m, 3H), 3.03 (t, J=7 Hz, 2H), 2.75 (dd, J=9.5, 13.4 Hz, 1H); IR 1787, 1761, 1699, 1390, 1375, 1308, 1208, 1203, 746, 699 cm⁻¹; mp 102-104°C.

### B. [4R-[3(2R,3R)]]-4-Benzyl-3-[2-benzyl-3-(4-bromo-2-fluoro-phenyl)-3-hydroxypropionyl]-oxazolidin-2-one

To a solution of (*R*)-4-benzyl-3-(3-phenyl-propionyl)-oxazolidin-2-one (1064 g, 3.44 mol) in dichloromethane (5.6 L) at -5°C was added dibutylboron triflate (1133 g, 4.13 mol) over 20 minutes, followed by the addition of triethylamine (719 mL, 5.16 mol) while maintaining a reaction temperature of less than 5°C. This mixture was cooled to -70°C, and a solution of 4-bromo-2-fluoro-benzaldehyde (699 g, 3.44 mol) in dichloromethane (2 L) was added over 30 minutes. The mixture was allowed to warm to -10°C over 1 hour, at which point it was judged to be complete by thin layer chromatography (hexanes/ethyl acetate, 2:1). The reaction was quenched by adding potassium phosphate monobasic-sodium hydroxide pH 7 buffer (3.5 L) over 30 minutes followed by methanol (1.8 L) and 35% aqueous hydrogen peroxide (1.8 L) over 1.5 hours while maintaining a reaction temperature of less than 15°C. The organic layer was separated, washed with saturated aqueous sodium bicarbonate (6.7 L), and diluted with anhydrous ethanol (4 L) and 25% aqueous sodium bisulfite. The organic layer was separated, washed with water (4 L), dried over magnesium sulfate, and concentrated *in vacuo* giving 1818 g (103% - crude weight) of [4*R*-[3(2*R*,3*R*)]]-4-benzyl-3-[2-benzyl-3-(4-bromo-2-fluoro-phenyl)-3-hydroxy-propionyl]-oxazolidin-2-one as a very viscous amber-colored oil: ¹H NMR (400 MHz, CDCl₃) δ 7.46 (t, J=8.0 Hz, 1H), 7.16-7.32 (m, 10H), 6.94-6.96 (m, 2H), 5.35 (d, J=4.7 Hz, 1H), 4.92-5.29 (m, 1H), 4.45-4.51 (m, 1H), 3.92 (m, 2H), 3.01-3.14 (m, 3H), 2.83 (dd, J=3.1, 13.6 Hz, 1H), 2.05 (dd, J=10.0, 13.5 Hz, 1H); IR 3460 (br), 1780, 1696, 1483, 1388, 1350, 1209, 1106, 1068, 877, 760, 747, 701, 583, 512, 486 cm⁻¹.

### C. [4R-[3(2R,3R)]]-4-Benzyl-3-[2-benzyl-3-(4-bromo-2-fluoro-phenyl)-3-hydroxypropionyl]-oxazolidin-2-one, 1-Methyl-2-pyrrolidinone Solvate

To a solution of (*R*)-4-benzyl-3-(3-phenyl-propionyl)-oxazolidin-2-one (12.0 kg, 38.8 mol) in dichloromethane (180 L) at -70°C to -80°C was added titanium tetrachloride (8.8 kg, 46.6 mol) over 30 minutes giving a thick suspension which was stirred for an additional 30 minutes at -70°C to -80°C. *N*,*N*,*N*',*N*'-Tetramethylethylenediamine (17.6 L, 116.4 mol) was added over 30 minutes giving a more fluid reaction mixture. 1-Methyl-2-pyrrolidinone (7.6 kg, 77.6 mol) was added, and the reaction mixture was stirred for 30 minutes, all while maintaining a reaction temperature of less than -65°C. A solution of 4-bromo-2-fluoro-benzaldehyde (7.9 kg, 38.8 mol) in dichloromethane (38 L) was added over 30 minutes while maintaining a reaction temperature of less than or equal to -68°C. The reaction mixture was allowed to warm to 20°C over 8 hours at which point it was cooled to 10°C and quenched with a solution of 5.0 kg of ammonium chloride in 11 L of water inducing a white precipitate and an exotherm to 28°C. Celite® (12 kg) was added and the reaction mixture was stirred for 12 hours at 20°C. The reaction mixture was filtered, concentrated atmospherically to an oil, treated with hexanes (120 L), concentrated to approximately 50 L, slowly cooled to 0°C and granulated for 24 hours. The crude product, 24.3 kg, was isolated by filtration, combined with the crude products from two similar reactions in 110 L of dichloromethane, treated with 320 L of hexanes, concentrated atmospherically to a final volume of approximately 250 L (distillate temperature 65°C), seeded with authentic product, and slowly cooled with granulation over 18 hours at 20°C. Filtration yielded 67.4 kg (94%) of [4*R*-[3(2*R*,3*R*)]]-4-benzyl-3-[2-benzyl-3-(4-bromo-2-fluoro-phenyl)-3-hydroxy-propionyl]-oxazolidin-2-one, 1-methyl-2-pyrrolidinone solvate as a light tan granular solid: ¹H NMR (400 MHz, CDCl₃) δ 7.46 (t, J=8.0 Hz, 1H), 7.15-7.29 (m, 10H), 6.94 (dd, J=1.9, 7.2 Hz, 2H), 5.34 (d, J=4.8 Hz, 1H), 4.91-4.96 (m, 1H), 4.44-4.49 (m, 1H), 3.90-3.95 (m, 2H), 3.55 (bs, 1H), 3.37 (dd, J=7.2, 7.2 Hz, 2H), 3.00-3.13 (m, 2H), 2.83 (s, 3H), 2.82 (dd, J=3.3, 13.3 Hz, 1H), 2.36 (dd, J=8.2, 8.2 Hz, 2H), 1.97-2.06 (m, 3H); IR 3150 (br), 1776, 1695, 1652, 1600, 1221, 1050, 996, 953, 875 cm⁻¹; mp 80-83°C.

### D. (1R,2S)-2-Benzyl-1-(4-bromo-2-fluoro-phenyl)-propane-1,3-diol

A 2 M solution of lithium borohydride in tetrahydrofuran (1.7 L, 3.4 mol) was diluted with tetrahydrofuran (1.7 L) and cautiously treated with water (61 mL, 3.4 mol) over 15 minutes. This mixture was stirred at room temperature until hydrogen evolution ceased (0.5 to 1 hour), and then added to a solution of [4*R*-[3(2*R*,3*R*)]]-4-benzyl-3-[2-benzyl-3-(4-bromo-2-fluoro-phenyl)-3-hydroxy-propionyl]-oxazolidin-2-one (1.75 kg, 3.4 mol) in tetrahydrofuran (8.75 L) at 0°C over 30 minutes. The resulting milky-white suspension was allowed to warm to room temperature over 12 hours at which point it was judged to be complete by thin layer chromatography (hexanes/ethyl acetate, 2:1). The reaction mixture was cooled to 15°C and quenched with water (5.25 L) over 15 minutes and stirred an additional 10 minutes before adding 35% aqueous hydrogen peroxide (2.6 L) over 20 minutes. The reaction mixture was stirred for 15 minutes and then diluted with ethyl acetate (5.3 L) and water (4 L). The organic layer was separated and washed with water (5.3 L), 5% aqueous sodium bisulfite (5.25 L), and 50% saturated aqueous sodium chloride (7.5 L). Peroxides were detected in the organic layer, so it was further washed with 5% aqueous sodium bisulfite (5 L) and 50% saturated aqueous sodium chloride (6 L). The organic layer was concentrated *in vacuo* to an oil, diluted with ethyl acetate (4 L) and hexanes (13 L), and washed with 1 N aqueous hydrochloric acid (6 times 17 L) to remove the (*R*)-(+)-4-benzyl-2-oxazolidinone. The organic layer was washed with saturated aqueous sodium bicarbonate (5.3 L), diluted with toluene (2 L), and concentrated *in vacuo* yielding 1138 g (98%) of (1*R*,2*S*)-2-benzyl-1-(4-bromo-2-fluoro-phenyl)-propane-1,3-diol as an oil: ¹H NMR (400 MHz, CDCl₃) δ 7.47-7.51 (m, 1H), 7.33 (dd, J=1.9, 8.3 Hz, 1H), 7.15-7.25 (m, 4H), 7.04-7.06 (m, 2H), 5.39 (d, J=2.6 Hz, 1H), 3.77 (dd, J=3.0, 10.7 Hz, 1H), 3.64 (dd, J=5.0, 10.8 Hz, 1H), 3.44 (bs, 1H), 2.68 (dd, J=11.0, 13.8 Hz, 1H), 2.59 (dd, J=4.1, 13.9 Hz, 1H), 2.15-2.20 (m, 1H), 2.01 (bs, 1H); IR 3370 (br), 3269 (br), 1485, 1406, 1213, 1033, 1021, 870, 700 cm⁻¹.

### E1. (3S,4R)-3-Benzyl-7-bromo-3,4-dihydro-benzopyran-4-ol

A 1 M solution of sodium bis(trimethylsilyl)amide in tetrahydrofuran (6.55 L, 6.55 mol) was added over 20 minutes to a solution of (1*R*,2*S*)-2-benzyl-1-(4-bromo-2-fluoro-phenyl)-propane-1,3-diol (1975 g, 5.82 mol) in dimethyl sulfoxide (9.88 L) at room temperature. The mixture was slowly heated to 60°C under aspirator vacuum to displace the tetrahydrofuran from the reaction mixture, and then heated at 60°C to 65°C for 5 hours under aspirator vacuum at which point the reaction was judged to be complete according to thin layer chromatography (hexanes/ethyl acetate, 2:1). The reaction mixture was cooled to room temperature and quenched by adding water (10 L) followed by 1 N aqueous hydrochloric acid (10 L). The resulting tan suspension was filtered, washed with water (2 L), and dissolved in ethyl acetate (12 L). This solution was washed with water (two times 12 L), concentrated to a low volume, dissolved in isopropyl ether (4 L), and concentrated under atmospheric pressure at 50 to 60°C to 1.0 L, at which point solids began to precipitate. The resulting suspension was cooled to room temperature, stirred for 12 hours, concentrated to one-half its volume, cooled to 0 to 5°C, and filtered giving 916 g (49%) of (3*S*,4*R*)-3-benzyl-7-bromo-3,4-dihydro-benzopyran-4-ol as a white solid. The filtrate was concentrated to a dark oil (906 g), dissolved in isopropyl ether (1.5 L) at reflux, cooled to room temperature, stirred, and filtered yielding an additional 82 g of solid. The filtrate was concentrated and chromatographed on silica gel (60-230 mesh) eluting with 3:1 hexanes/ethyl acetate. Product-rich fractions were concentrated and recrystallized from isopropyl ether yielding an additional 82 g of solid. The total yield of (3*S*,4*R*)-3-benzyl-7-bromo-3,4-dihydro-benzopyran-4-of was 1080 g (58%): ¹H NMR (400 MHz, CDCl₃) δ 7.29-7.33 (m, 2H), 7.21-7.25 (m, 1H), 7.15-7.19 (m, 3H), 7.06-7.09 (m, 2H), 4.44 (bs, 1H), 4.21 (dd, J=2.6, 11.3 Hz, 1H), 3.97 (dd, J=4.5, 11.3 Hz, 1H), 2.68 (dd, J=6.5, 13.8 Hz, 1H), 2.51 (dd, J=9.1, 13.8 Hz, 1H), 2.18-2.23 (m, 1H), 1.85 (d, J=4.3 Hz, 1H); IR 3274 (br), 3181 (br), 1598, 1573, 1493, 1480, 1410, 1219, 1070, 1052, 1023, 859, 700 cm⁻ ¹; mp 143.5-144.0°C.

### E2. (3S,4R)-3-Benzvl-7-bromo-3,4-dihvdro-benzopyran-4-ol

Alternatively, the above named compound can be prepared as follows: To a solution of (1*R*,2*S*)-2-benzyl-1-(4-bromo-2-fluoro-phenyl)-propane-1,3-diol (prepared from 33.5 kg (54.8 moles) of [4*R*-[3(2*R*,3*R*)]]-4-benzyl-3-[2-benzyl-3-(4-bromo-2-fluoro-phenyl)-3-hydroxy-propionyl]-oxazolidin-2-one, 1-methyl-2-pyrrolidinone solvate without isolation) in 185 L of tetrahydrofuran was added 12.9 kg (115 mol) of potassium *tert*-butoxide. The reaction mixture was heated at reflux for 4 hours at which point the reaction was found to be complete by thin layer chromatography (hexanes/ethyl acetate, 3:1). The reaction mixture was cooled to room temperature, quenched with 170 L of water, diluted with 83 L of ethyl acetate, and acidified to pH 5.3 (aqueous layer) with 7.5 L of concentrated hydrochloric acid. The organic layer was concentrated under vacuum to approximately 38 L of a slurry, diluted with 76 L of isopropyl ether, warmed to dissolve the solids, slowly cooled to 0°C, and granulated at 0°C for 12 hours. (3*S*,4*R*)-3-Benzyl-7-bromo-3,4-dihydro-benzopyran-4-ol, 5.1 kg of white solid, was isolated by filtration. The mother liquor was washed with 4 L of saturated aqueous sodium chloride, concentrated to a final volume of 57 L, and granulated at 0°C for 12 hours affording a 4.3 kg second crop of (3*S*,4*R*)-3-benzyl-7-bromo-3,4-dihydro-benzopyran-4-ol.

A second identical reaction mixture was quenched, diluted with ethyl acetate, and acidified as described above. The organic layer was dried over 10 kg of magnesium sulfate, concentrated atmospherically to approximately 30 L of a slurry, diluted with 38 L of isopropyl ether, concentrated to approximately 57 L, slowly cooled, and granulated at 0 to 10°C for 12 hours. (3*S*,4*R*)-3-Benzyl-7-bromo-3,4-dihydro-benzopyran-4-ol, 8.7 kg, was isolated by filtration. The mother liquor was combined with the mother liquor from the second crop from the first reaction, concentrated to an oil, solidified by cooling, granulated in 6 L of isopropyl ether at 20°C for 12 hours and 0°C for 2 hours, and filtered giving 6.3 kg of (3*S*,4*R*)-3-benzyl-7-bromo-3,4-dihydro-benzopyran-4-ol after washing with cold isopropyl ether. The combined crops from both reactions were dried giving 20.8 kg (59%) of (3*S*,4*R*)-3-benzyl-7-bromo-3,4-dihydro-benzopyran-4-ol.

### F. (3S,4R)-(3-Benzyl-4-hvdroxv-3,4-dihydro-benzopyran-7-yl)-boronic Acid

To a solution of (3*S*,4*R*)-3-benzyl-7-bromo-3,4-dihydro-benzopyran-4-ol (377 g, 1.18 mol) in tetrahydrofuran (5.6 L) at -75°C was added a 1.48 M solution of methyllithium in ether (1.6 L, 2.37 mol) over 45 minutes while maintaining a temperature of less than -65°C. The reaction mixture was stirred at less than -65°C for 1 hour, followed by the addition of a 2.5 M solution of butyllithium in hexanes (440 mL, 1.3 mol) over 15 minutes. The reaction mixture was stirred at less than -65°C for 1 hour, followed by the addition of a 1.0 M solution of borane-tetrahydrofuran complex in tetrahydrofuran (5.9 L, 5.9 mol) over 30 minutes. The reaction mixture was warmed to 0°C, quenched by adding water (4.4 L), adjusted to pH 2 with 1 N aqueous hydrochloric acid (4 L), and extracted with isopropyl ether (4 L). The aqueous layer was extracted with isopropyl ether (4 L), and the combined organic layers were washed with 0.5 N aqueous sodium hydroxide (7.2 L). The aqueous layer was adjusted to pH 3 with 1 N aqueous hydrochloric acid (5.5 L) and extracted with ethyl acetate (5.4 L and 2.7 L). The combined ethyl acetate layers were dried over magnesium sulfate, and concentrated *in vacuo* yielding 304.5 g (91%) of (3*S*,4*R*)-(3-benzyl-4-hydroxy-3,4-dihydro-benzopyran-7-yl)-boronic acid as a yellow foam: ¹H NMR (300 MHz, CDCl₃) δ 7.35-7.00 (m, 8H), 4.42 (d, J=4.1 Hz, 1H), 4.19 (d, J=11 Hz, 1H), 3.90 (m, 1H), 2.68 (dd, J=6.2, 13.8 Hz, 1H), 2.47 (m, 1H), 2.15 (m, 1H); IR 3330 (br), 1413, 1348, 1320, 1211, 1025, 749, 730, 700 cm⁻¹.

### G. (3S,4R)-2-(3-Benzyl-4-hydroxy-3,4-dihydro-benzopyran-7-yl)-4-trifluoromethyl-benzoic Acid Ethyl Ester

A mixture of ethyl 2-iodo-4-trifluoromethyl-benzoate (723 g, 2.1 mol), (3*S*,4*R*)-(3-benzyl-4-hydroxy-3,4-dihydro-benzopyran-7-yl)-boronic acid (627 g, 2.2 mol), potassium fluoride (366 g, 6.3 mol), 10% palladium on carbon (157 g, 50% water wet), and anhydrous ethanol (6.27 L) was heated at reflux for 3 hours at which point thin layer chromatography (toluene/acetic acid, 5:1) indicated the reaction to be complete. The reaction mixture was diluted with isopropyl ether (8 L), filtered through Celite® and washed with 10% aqueous sodium bicarbonate (1.5 L). The aqueous layer was separated and extracted with isopropyl ether (3 L). The combined organic layers were washed with water (6 L), dried over magnesium sulfate, and treated with Darco® G-60 (1.0 kg) and silica gel (1 kg, 70-230 mesh) at room temperature. This mixture was filtered through a pad of silica gel (70-230 mesh) and concentrated *in vacuo* to 922 g of dark oil. This oil was diluted with ethyl acetate (1 L) and filtered through a column of silica gel (2 kg) eluting with ethyl acetate giving a light amber solution which was concentrated to afford 897 g (92%) of (3*S*,4*R*)-2-(3-benzyl-4-hydroxy-3,4-dihydro-benzopyran-7-yl)-4-trifluoromethyl-benzoic acid ethyl ester as a light amber oil: ¹H NMR (400 MHz, CDCl₃) δ 7.89 (d, J=8.1 Hz, 1H), 7.63-7.67 (m, 2H), 7.18-7.38 (m, 6H), 6.91 (dd, J=1.8, 7.8 Hz, 1H), 6.86 (d, J=1.7 Hz, 1H), 4.55 (bs, 1H), 4.25 (dd, J=2.7, 11.2 Hz, 1H), 4.17 (q, J=7.1 Hz, 2H), 4.00 (ddd, J=1.0, 4.5, 11.2 Hz, 1H), 2.75 (dd, J=6.4, 13.9 Hz, 1H), 2.56 (dd, J=9.3, 13.8 Hz, 1H), 2.26 (m, 1H), 1.93 (d, J=4.3 Hz, 1H), 1.09 (t, J=7.2 Hz, 3H); IR 3307 (br), 3216 (br), 1734, 1339, 1298, 1247, 1191, 1175, 1118, 1097, 1050 cm⁻¹.

### PREPARATION 2

### (3S,4R)-2-(3-Benzyl-4-hydroxy-3,4-dihydro-benzopyran-7-yl)-4-trifluoromethylbenzoic Acid

A solution of (3S,4R)-2-(3-benzyl-4-hydroxy-3,4-dihydro-benzopyran-7-yl)-4-trifluoromethyl-benzoic acid 2,2-dimethyl-propyl ester (2.34 g, 4.69 mmol) in isopropyl alcohol (23 mL) was treated with 10% aqueous sodium hydroxide (2.3 mL, 6.4 mmol) and heated at reflux for 3 hours. The reaction mixture was cooled to room temperature, poured into water (34 mL), and extracted with hexanes (23 mL) and isopropyl ether (13 mL). The aqueous layer was separated and extracted with hexanes (23 mL) and isopropyl ether (13 mL), adjusted to pH 2 with 6N aqueous hydrochloric acid, and extracted with ethyl acetate (two times 40 mL). The combined ethyl acetate extracts were washed with brine (40 mL), dried over magnesium sulfate, filtered and concentrated to a white foam which was recrystallized from toluene/hexanes. The resulting solids were filtered and washed with hexanes, and the wet cake was stirred with hexanes (20 mL) for 1 hour. The mixture was filtered, and the resulting solids were dried under vacuum to provide 1.01 g (50% yield) of (3S,4R)-2-(3-benzyl-4-hydroxy-3,4-dihydro-benzopyran-7-yl)-4-trifluoromethyl-benzoic acid as a white solid: ¹H NMR (400 MHz, CDCl₃) δ 8.00 (d, J = 8.1 Hz, 1H), 7.67 (d, J = 8.1 Hz, 1H), 7.64 (s, 1H), 7.18-7.36 (m, 6H), 6.91 (dd, J = 7.9, 1.7 Hz, 1H), 6.86 (d, J = 1.7 Hz, 1H), 4.53 (d, J = 4.2 Hz, 1H), 4.24 (dd, J = 11.2, 2.7 Hz, 1H), 3.97 (dd, J = 11.0, 4.0 HZ, 1H), 2.76 (dd, J = 13.9, 6.4 Hz, 1H), 2.53 (dd, J = 13.7, 9.3 Hz, 1H), 2.24-2.26 (m, 1H).

The (3S,4R)-2-(3-benzyl-4-hydroxy-3,4-dihydro-benzopyran-7-yl)-4-trifluoromethylbenzoic acid 2,2-dimethyl-propyl ester starting material for preparation 2 was prepared as follows:

### A. 4-Trifluoromethyl-benzoic Acid 2,2-Dimethyl-propyl Ester

To a suspension of 4-trifluoromethylbenzoic acid (75.0 g, 394 mmol) and 2,2-dimethyl-propyl alcohol (70.5 g, 800 mmol) in toluene (500 mL) was added concentrated sulfuric acid (3.0 mL). The mixture was stirred at reflux for 4 hours, cooled to room temperature, poured into saturated aqueous sodium carbonate (250 mL) and the layers were separated. The organic layer was washed with saturated aqueous sodium carbonate (250 mL), and brine (100 mL), and was concentrated to give 4-trifluoromethyl-benzoic acid, 2,2-dimethyl-propyl ester (102 g, 99% yield) as a yellow liquid: R_{f}: 0.66 (ethyl acetate/hexanes 25/75); IR 2932, 1727, 1327, 1280, 1133, 1066, 862, 775, 704 cm⁻¹; ¹H NMR (400 MHz, CDCl₃) δ 8.16 (d, J = 7.9 Hz, 2H), 7.70 (d, J = 8.1 Hz, 2H), 4.04 (s, 2H), 1.04 (s, 9); ¹³C NMR (100 MHz, CDCl₃) δ 26.51, 31.61, 74.72, 123.63 (q, J = 272.7 HZ), 125.4, 129.9, 133.7, 134.35 (q, J = 31.7 Hz), 165.35.

### B. 2-(2,2-Dimethyl-propoxycarbonyl)-5-trifluoromethyl-benzeneboronic Acid

To a solution of 4-trifluoromethyl-benzoic acid 2,2-dimethyl-propyl ester (4.225 g, 16.23 mmol) in tetrahydrofuran (40 mL) was added triisopropylborate (9.00 mL, 39.0 mmol). The solution was cooled to -78°C and lithium diisopropylamide (12.0 mL of a 2.0 M solution in tetrahydrofuran/heptane, 24.0 mmol) was added dropwise over 5 minutes. The red solution was stirred for 30 minutes, warmed to 0°C, and quenched by the slow addition of 1N hydrochloric acid (50 mL). The mixture was allowed to warm to room temperature, stirred for 30 minutes and added to hexanes (200 mL). The layers were separated and the organic layer was washed successively with 2N hydrochloric acid (two times with 100 mL), water (100 mL), and brine (50 mL). The organic extracts were dried over magnesium sulfate, filtered, and concentrated to an oil. The crude product was crystallized from heptane (40 mL) to provide 2-(2,2-dimethyl-propoxycarbonyl)-5-trifluoromethyl-benzeneboronic acid (3.037 g, 62% yield) as a white solid: mp = 159-160°C; IR 3377 (br), 2963, 1703, 1371, 1308, 1171, 1131, 794, 709 cm⁻¹; ¹H NMR (400 MHz, DMSO/D₂O) δ 8.05 (d, J = 8.1 Hz, 1H), 7.78 (d, J = 8.3 Hz, 1H), 7.66 (s, 1H), 3.94 (s, 2H), 0.95 (s, 9H); ¹³C NMR (100 MHz, DMSO/D₂O) δ 26.69, 31.69, 74.91, 125.29, 125.75, 128.30, 129.62, 131.98 (q, J = 31.8 Hz), 136.28, 142.68, 166.90.

### C. (3S,4R)-2-(3-Benzyl-4-hydroxy-3,4-dihydro-benzopyran-7-yl)-4-trifluoromethyl-benzoic Acid 2,2-Dimethyl-propyl Ester

A bi-phasic solution of 2-(2,2-dimethyl-propoxycarbonyl)-5-trifluoromethylbenzeneboronic acid (1.72 g, 5.66 mmol), (3S,4R)-3-benzyl-7-bromo-3,4-dihydrobenzopyran-4-ol (1.80 g, 5.63 mmol), sodium carbonate (1.82 g, 17.2 mmol), and tretrakis(triphenyl-phosphine)palladium(0) (12 mg, 0.19 mol%) in toluene (15 mL) and water (9 mL) was stirred at reflux for 100 minutes. The reaction mixture was cooled to room temperature, poured into water (40 mL) and extracted with diisopropylether (75 mL). The organic extracts were washed with brine (50 mL), treated with Darco® G-60, dried over magnesium sulfate, filtered through Celite®, and concentrated. the crude product was purified by chromatography on silica gel (ethyl acetate/hexanes 20/80) to provide (3S, 4R)-2-(3-benzyl-4-hydroxy-3,4-dihydro-benzopyran-7-yl)-4-trifluoromethyl-benzoic acid 2,2-dimethylpropyl ester as a white foam (2.35 g, 84% yield): R_{f}: 0.32 (ethyl acetate/hexanes 25/75); IR 3407 (br), 2961, 1721, 1336, 1292, 1252, 1172, 1134, 1110, 1022, 848, 749 cm⁻¹; ¹H NMR (400 MHz, CDCl₃) δ 7.90 (d, J = 8.1 Hz, 1H), 7.66 (d, J = 8.1 Hz, 1H), 7.63 (s, 1H), 7.19-7.37 (m, 6H), 6.88-6.93 (m, 2H), 4.53 (t, J = 4.4 Hz, 1H), 4.22 (dd, J = 11.2, 2.5 Hz, 1H), 3.99 (dd, J = 11.2, 3.3 Hz, 1H), 3.78 (s, 2H), 2.73 (dd, J = 13.8, 6.3 Hz, 1H), 2.54 (dd, J = 13.6, 9.4 Hz, 1H), 2.20-2.80 (m, 1H), 1.81 (d, J = 5.2 Hz, 1H), 0.74 (s, 9H); ¹³C NMR (100 MHz, CDCl₃) δ 26.64, 30.96, 34.62, 41.53, 64.76, 67.42, 75.33, 116.77, 121.07, 122.97, 124.13, 126.44, 127.50, 127.54, 128.45, 128.60, 128.92, 129.11, 130.25, 130.31, 139.08, 141.69, 142.03, 154.44, 168.14.

### PREPARATION 3

### (3S,4R)-2-(3-Benzyl-4-hydroxy-3,4-dihydro-benzopyran-7-yl)-4-trifluoromethylbenzoic Acid

A mixture of (3*S*,4*R*)-dicyclohexylammonium-2-(3-benzyl-4-hydroxy-3,4-dihydrobenzopyran-7-yl)-4-trifluoromethyl-benzoate (2.37 g, 3.89 mmol) in ethyl acetate (25 mL), and 1 N hydrochloric acid (25 mL) was stirred at room temperature for 1 hour. The mixture was poured into ethyl acetate (20 mL) and the aqueous layer was removed. The organic layer was washed with water (six times 50 mL), dried over magnesium sulfate, filtered, and concentrated to provide (3*S*,4*R*)-2-(3-benzyl-4-hydroxy-3,4-dihydro-benzopyran-7-yl)-4-trifluoromethyl-benzoic acid (1.66 g, 100% yield): ¹H NMR (400 MHz, CDCl₃) δ 8.00 (d, J = 8.1 Hz, 1H), 7.67 (d, J = 8.1 Hz, 1H), 7.64 (s, 1H), 7.18-7.36 (m, 6H), 6.91 (dd, J = 7.9, 1.7 Hz, 1H), 6.86 (d, J = 1.7 Hz, 1H), 4.53 (d, J = 4.2 Hz, 1H), 4.24 (dd, J = 11.2, 2.7 Hz, 1H), 3.97 (dd, J = 11.0, 4.0 Hz, 1H), 2.76 (dd, J = 13.9, 6.4 Hz, 1H), 2.53 (dd, J = 13.7, 9.3 Hz, 1H), 2.24-2.26 (m, 1H).

The (3*S*,4*R*)-dicyclohexylammonium-2-(3-benzyl-4-hydroxy-3,4-dihydro-benzopyran-7-yl)-4-trifluoromethyl-benzoate starting material of preparation 3 was prepared as follows:

### A. 2-[1,3,6,2]Dioxazaborocan-2-yl-4-trifluoromethyl-benzoic acid 2,2-dimethyl-propyl Ester

To a solution of 4-trifluoromethyl-benzoic acid 2,2-dimethyl-propyl ester (35.8 g, 138 mmol) in tetrahydrofuran (250 mL) was added triisopropylborate (73.0 mL, 316 mmol). The solution was cooled to 0°C, lithium diisopropylamide (73.0 mL of a 2.0 M solution in tetrahydrofuran/heptane, 146.0 mmol) was added dropwise over 20 minutes, and the red solution was stirred for an additional 30 minutes. Hexanes (200 mL) was added followed by 1*N* hydrochloric acid (200 mL). The mixture was stirred for 10 minutes and poured into hexanes (200 mL). The organic layer was washed with 1 N hydrochloric acid (two times 150 mL), and brine (100 mL). The organic extracts were dried over magnesium sulfate, filtered, and concentrated to about 200 mL. Isopropyl alcohol (100 mL), and diethanolamine (15.95 g, 151.7 mmol) were added, and the mixture was stirred at room temperature for 10 hours. The solids were filtered and washed with a mixture of isopropyl alcohol (15 mL) and hexanes (30 mL) to provide 2-[1,3,6,2]dioxazaborocan-2-yl-4-trifluoromethyl-benzoic acid 2,2-dimethyl-propyl ester (37.83 g, 74% yield) as a white solid. mp = 233-234 °C; IR 3077, 2963, 2862, 1722, 1480, 1467, 1371, 1331, 1298, 1290, 1279, 1254, 1161, 1117, 1108, 1087, 1074, 995, 952, 862, cm⁻¹; ¹H NMR (400 MHz, CDCl₃) d 8.23 (s, 1H), 7.72 (d, J = 7.9 Hz, 1H), 7.52 (dd, J = 7.9, 1.3 Hz, 1H), 6.33 (brs, 1H), 4.08-4.14 (m, 2H), 3.98 (s, 2H), 3.93-3.98 (m, 2H), 3.42-3.50 (m, 2H), 2.88-2.94 (m, 2H), 1.02 (s, 9H); ¹³C NMR (100 MHz, CDCl₃) 26.51, 31.69, 50.92, 63.33, 74.72, 123.94, 128.59, 132.06, 139.61, 171.56.

### B. (3S,4R)-Dicyclohexylammonium-2-(3-benzvl-4-hydroxy-3,4-dihydrobenzopyran-7-yl)-4-trifluoromethyl-benzoate

A mixture of 2-[1,3,6,2]dioxazaborocan-2-yl-4-trifluoromethyl-benzoic acid 2,2-dimethyl-propyl ester (7.04 g, 18.9 mmol) in toluene (45 mL) and 1.5 N hydrochloric acid (45 mL) was stirred at room temperature for 45 minutes. The aqueous layer was removed and sodium carbonate (2.73 g, 25.8 mmol), (3*S*,4*R*)-3-benzyl-7-bromo-3,4-dihydrobenzopyran-4-ol (5.47 g, 17.1 mmol), tetrakis(triphenylphosphine)palladium(0) (24.0 mg, 20.8 µmol), and water (20 mL) were added. The bi-phasic solution was stirred at reflux for 100 minutes, cooled to room temperature, and poured into water (50 mL). The layers were separated, and the organic layer was treated with Darco® G-60, filtered, and concentrated. The crude ester was dissolved in isopropyl alcohol (80 mL) and 10% aqueous sodium hydroxide (8.0 mL) was added. The solution was heated at reflux for 3 hours, cooled to room temperature, poured into water (120 mL), and extracted with hexanes (80 mL) and isopropyl ether (40 mL). The aqueous layer was washed with hexanes (80 mL) and isopropyl ether (40 mL), adjusted to pH 2 with 6 N hydrochloric acid, and extracted with methyl *tert*-butyl ether (two times 75 mL). The organic extracts were dried over magnesium sulfate, filtered, and concentrated. The crude product was dissolved in methyl *tert*-butyl ether (40 mL), and dicyclohexylamine (4.10 mL, 20.6 mmol) was added. The mixture as stirred overnight, and the solid was filtered and washed with methyl *tert*-butyl ether (20 mL) to afford (3*S*,4*R*)-dicyclohexylammonium-2-(3-benzyl-4-hydroxy-3,4-dihydro-benzopyran-7-yl)-4-trifluoromethylbenzoate (7.32 g, 70% yield): mp = 209-210 °C; IR 3307, 3025, 2939, 2858, 1626, 1564, 1429, 1398, 1388, 1333, 1168, 1119, 903, 875, 846, 838 cm⁻¹; ¹H NMR (400 MHz, CDCI₃) δ 7.62 (d, J = 7.7 Hz, 1H), 7.55 (s, 1H), 7.52 (d, J = 7.9 Hz, 1H), 7.17-7.31 (m, 6H), 7.08 (dd, J = 7.9, 1.7 Hz, 1H), 7.00 (d, J = 1.7 Hz, 1H), 4.48 (d, J - 4.4 Hz, 1H), 4.17 (dd, J = 11.0, 2.6 Hz, 1H), 3.90 (dd, J = 11.0, 5.0 Hz 1H), 2.74-2.79 (m, 3H), 2.50 (dd, J = 13.8, 9.4 Hz, 1H), 1.80-1.82 (m, 4H), 2.20 (brs, 1H), 1.68-1.70 (m, 4H), 1.56 (d, J = 12.2 Hz, 2H), 1.00-1.26 (m, 10H). ¹³C NMR (100 MHz, CDCl₃) δ 24.70, 24.73, 25.03, 28.94, 29.09, 34.75, 41.75, 52.64, 65.00, 67.57, 116.50, 121.42, 122.59, 123.77, 126.38, 126.73, 128.03, 128.55, 129.06, 129.45, 138.95, 139.16, 142.51, 144.20, 154.04, 173.85.

### PREPARATION 4

### 3S,4R-7-(3-carboxyphenyl)-4-hydroxy-3-phenylmethyl-3,4-dihydro-2H-1-benzopyran

Saponification: To a stirred solution of the less polar 4R,3S N-α-t-Butoxycarbonyl-L-tryptophan-7-[(3-carbomethoxyphenyl)-3-phenylmethyl]-3,4-dihydro-benzopyran-4-yl]-ester (840 mg, 1.08 mmole) in 10 mL of methanol was added 10 mL of 2M NaOH solution. The mixture was refluxed for 8 hours, cooled and acidified to a pH of 4 with 1 M HCI. The cloudy emulsion was extracted with 3 times 20 mL of ethyl acetate, and the combined organic fractions were washed with brine and dried over MgSO₄. Filtration and solvent removal in vacuo afforded a yellow foam. Chromatography (silica gel-ethyl acetate: hexane: acetic acid - 35:75:1) afforded 210 mg of product. ¹H NMR. (300 MHz, CD₃ CN): 8.22 (1 H, t, 1.7 Hz), 7.97 (1 H, dt, J=7.8, 1.7 Hz), 7.87 (1 H, dt, J=7.8, 1.7 Hz), 7.55 (1 H, t, J=7.8 Hz), 7.42 (1 H, d, J=7.9 Hz), 7.15-7.36 (6 H, m), 7.10 (1 H, d, J=1.8 Hz), 4.44 (1 H, d, J=4.9 Hz), 4.19 (1 H, dd, J=9.1, 2.5 Hz), 3.97 (1 H, dd, J=9.1, 5.4 Hz), 2.72 (1 H, dd, J=13.7, 6.2 Hz), 2.51 (1 H, dd, J=13.7, 9.1 Hz), 2.04-2.20 (3 H, m). [α]_{D}²⁵=+11.1 at C=1.00 in methanol. M.P.=210-212°C. Saponification as above of the more polar 3R,4S tryptophan-ester (700 mg) gave the 3R,4S enantiomer, ¹H-NMR (300 MHz, CD₃CN): 8.22 (1 H, t, 1.7 Hz), 7.97 (1 H, dt, J=7.8, 1.7 Hz), 7.87 (1 H, dt, J=7.8, 1.7 Hz), 7.55 (1 H, t, J=7.8 Hz), 7.42 (1 H, d, J=7.9 Hz), 7.15-7.36 (6 H, m), 7.10 (1 H, d, J=1.8 Hz), 4.44 (1 H, d, J=4.9 Hz),4.19 (1 H, dd, J=9.1, 2.5 Hz), 3.97 (1 H, dd, J=9.1, 5.4 Hz), 2.72 (1 H, dd, J=13.7, 6.2 Hz), 2.51 (1 H, dd, J=13.7, 9.1Hz), 2.04-2.20 (3 H, m). [α]_{D}²⁵= -11.0 at c=1.01 in methanol. mp 209- 211°C.

### Trans-3-phenylmethyl-4-hydroxy-7-(3-carboxyphenyl)-3,4-dihydro-2H-1-benzopyran

Saponification as described above of the trans ring isomer gave the corresponding acid. ¹H NMR (300 MHz, CD₃ CN): 8.22 (1 H, t, 1.7 Hz), 7.97 (1 H, dt, J=7.8, 1.7 Hz), 7.87 (1 H, dt, J=7.8, 1.7 Hz), 7.55 (1 H, t, J=7.8 Hz), 7.42 (1 H, d, J=7.9 Hz), 7.15-7.36 (6 H, m), 7.10 (1 H, d, J=1.8 Hz), 4.44 (1 H, d, J4.9 Hz), 4.19 (1 H, dd, J=9.1, 2.5 Hz), 3.97 (1 H, dd, J=9.1, 5.4 Hz), 2.72 (1 H, dd, J=13.7, 6.2 Hz), 2.51 (1 H, dd, J=13.7, 9.1 Hz), 2.04-2.20 (3 H, m). M.P. 210-212°C.

The N-α-t-Butoxycarbonyl-L-tryptophan-7-[(3-carbomethoxyphenyl)-3-phenylmethyl]-3,4-dihydro-benzopyran-4-yl]-ester starting material for preparation 4 was prepared as follows:

### A. 2,4-Dihydroxy-3-chloropropiophenone

To a stirred mixture of resorcinol (200 g, 1.82 mol) and 3-chloropropionic acid (200 g, 1.84 mol) was added trifluoromethanesulfonic acid (1 kg) in one portion. The solution was heated slowly over 45 minutes to 80°C, then cooled to room temperature over 15 minutes and poured into chloroform (4.0 L). The organic portion was slowly poured into water (4.0 L) and the layers separated. The aqueous layer was extracted with chloroform (2 times 2.0 L). The combined organic layers were washed with brine, dried over sodium sulfate and filtered. Concentration in vacuo gave an orange semi-solid (244.1 g), which was used crude in the next step. ¹H-NMR (300 MHz, CDCl₃): 12.56 (1 H, s), 7.63 (1 H, d, J=7.6 Hz), 6.37-6.46 (2 H, m), 3.92 (2 H, t, J=6.3Hz), 3.41 (2 H, t, J=6.3 Hz).

### B. 7-Hydroxy-2,3-dihydro-benzopyran-4-one

To a cooled (5°C) solution of 2N sodium hydroxide (10.0 L) was added the compound of step A (244.1 g) in one portion. The solution was warmed to room temperature over 2 hours using a warm water bath then recooled to 50°C and the pH adjusted to 2 with 6M sulfuric acid (1.2 L). The mixture was extracted with 3 times 3.0 L of ethyl acetate, washed with brine (1 times 2.0 L) dried over sodium sulfate and filtered. Concentration in vacuo gave a tan solid. Trituration with hexanes, and filtration afforded 173.7 g (58% yield) of the title compound. M.P. 136°C-137°C.

### C. 7-[(Trifluoromethylsulfonyl)oxyl-2,3-dihydro-benzopyran-4-one

To a stirred solution of 7-hydroxy-2,3-dihydro-benzopyran-4-one (173.7 g, 1.05 mole) in methylene chloride (3.0 L) at -78°C. was added triethylamine (320 g, 3.16 mole) and dimethylaminopyridine (2.5 g). After total dissolution, trifluoromethane sulfonic anhydride (327 g, 1.16 mole) was added dropwise over 20 minutes, the material was stirred for 30 minutes at -78°C and then warmed to room temperature over 2 hours. The reaction mixture was poured into saturated ammonium chloride solution (2.5 L) and the layers separated. The aqueous layer was extracted with 2 times 2.0 L of methylene chloride. The combined organic fractions were washed with water (1 times 1.0 L), dried over magnesium sulfate and filtered. Concentration in vacuo gave a red oil. Chromatography over silica gel (1 kg) eluting with (8: 1) hexane: ethyl acetate gave after solvent removal 211.1 g. (69% yield) of the title product. mp. 43-44 °C.

### D. 7-[(Trifluoromethylsulfonyl)oxy]-3-phenylmethylidenyl-2,3-dihydrobenzopyran-4-one

To a stirred solution of 7-[(trifluoromethylsulfonyl)oxy]-2,3-dihydro-benzopyran-4-one (27 g, 91.2 mmole) in 183 mL of methanol was added benzaldehyde (11.1 mL, 109 mmole) followed by pyrrolidine (9.1 mL, 109 mmole). The mixture was stirred at room temperature ovemight, cooled to 0°C and filtered. The solid was washed once with 50 mL of ice-cold methanol and then dried in vacuo; 35.2 g, (75% yield) of the title product was recovered. mp. 133-135°C. ¹H NMR (300 MHz, CDCl₃): 8.11 (1 H. d, J=8.7 Hz), 7.91 (1 H, bs), 7.40-7.51 (2 H, m), 7.24-7.38 (3 H, m), 6.97 (1H, dd, J=8.7 Hz, 2.4 Hz), 6.91 (1 H, d, J=2.4 Hz), 5.40 (1 H, bs).

### E. 7-[(Trifluoromethylsulfonyl)oxy]-3-phenylmethyl-2,3-dihydro-benzopyran-4-one

To a solution of 7-[(trifluoromethylsulfonyl)oxy]-3-phenylmethylidenyl-2,3-dihydrobenzopyran-4-one (26.6 g, 69.2 mmole) in 250 mL of ethyl acetate in a 500 mL Parr shaker flask was added 10% palladium on carbon catalyst (1.3 g). The mixture was hydrogenated at 40 psi until hydrogen uptake ceased after about 3 hours. The mixture was filtered through CELITE® to remove the palladium catalyst, and chromatographed over silica gel (hexane-ether); 25.1 g (94% yield) of the title product was obtained. mp. 56-58°C. ¹H NMR (300 MHz, CDCI): 8.01 (1 H, d, J=8.5 Hz), 7.20-7.35 (5 H.sub., m), 6.981-6.96 (2 H, m), 4.42 (1 H, dd, J=11.6, 4.4 Hz), 4.22 (1 H, dd, J=11.6 Hz, 8.7 Hz), 3.26 (1 H, dd, J=14.0, 4.4 Hz), 2.90-3.05 (1 H, m), 2.70(1 H, dd, J=14.0, 8.7 Hz).

### F. 7-(Trimethylstannyl)-3-phenylmethyl-2,3-dihydro-benzopyran-4-one

To a stirred solution of 7-[(trifluoromethylsulfonyl)oxy]-3-phenylmethyl-2,3-dihydrobenzopyran-4-one (9.20 g, 25.0 mmole) in 200 mL of dioxane was added lithium chloride (3.20, 75.0 mmole), Pd(PPh₃), (1.15 9, 1.0 mmole), 3 crystals of butylated hydroxytoluene, and hexamethylditin (9.0 g, 27.5 mmole). The mixture was heated to reflux for 1.5 hours, cooled to room temperature and poured into 150 mL of saturated, aqueous ammonium chloride solution. The mixture was extracted with 3 times 150 mL of diethylether and the combined organic fractions were washed with brine, dried over sodium sulfate and filtered. Evaporation in vacuo gave a yellow semi solid which was chromatographed over silica gel (5:1 hexane: ether) to give 8.90 g (89% yield) of the title product. mp. 84°C-86°C. ¹H NMR (300 MHz,CDCl₃): 7.85 (1 H, d, J=8.7 Hz), 7.18-7.37 (5 H, m), 7.14 (1 H, d, J=8.7 Hz), 7.11 (1 H. s), 4.38(1 H, dd, J=1 1.6,4.5 Hz), 4.17 (1 H, dd, J=1 1.6 Hz, 8.4 Hz), 3.28 (1 H. dd, J=14.0, 4.4 Hz), 2.84-2.95 (1 H. m), 2.71 (1H. dd, J=l14 Hz, J=1 1.OHz), 0.31 (9 H, s).

### G. 7-(3-Carbomethoxyphenyl)-3-phenylmethyl-2,3-dihydro-benzopyran-4-one

To a stirred solution of 7-(trimethylstannyl)-3-phenylmethyl-2,3-dihydro-benzopyran-4-one (7.0 g, 17.5 mmole) in dimethylformamide (DMF) (35 mL) was added Pd(PPh₃)₂Cl₂(490 mg, 0.7 mmole), 3 crystals of BHT and methyl 3-iodobenzoate (5.0 g, 19.1 mmole). The mixture was stirred at reflux for 1.5 hours, cooled to room temperature and poured into 150 mL of saturated aqueous ammonium chloride solution. The mixture was extracted with 3 times 150 mL of diethyl ether, and the combined extract was washed with 2 times 1 00 mL of water, and then brine. The solution was dried over sodium sulfate, filtered and evaporated in vacuo to afford a yellow oil. Chromatography over silica gel (4:1 hexane: ether elution) afforded 6.51 g of the title compound as a viscous oil. ¹H NMR (300 MHz, CDCl₃): 8.29 (1 H, t, J=1.6 Hz), 8.06 (1 H, dd, J=7.6, 1.6 Hz), 8.00 (1 H, d, J=8.2 Hz), 7.79 (1 H, dd, J=7.6 Hz, 1.6 Hz), 7.53 (1 H, t, J=7.6 Hz), 7.22-7.36 (7 H, m), 4.41 (1 H, dd, J=11.6, 4.5 Hz), 4,21 (1 H, dd, J=11.6, 8.5 Hz), 3.94 (3 H, s), 3.31 (1 H, dd, J=14.0, 4.4hz), 2.91-2.99 (1 H, m), 2.73 (1 H, dd, J=14.0, 11.1 Hz)

### H. 7-(3-Carbomethoxyphenyl)-4-hydroxy-3-phenylmethyl-2,3-dihydrobenzopyran

To a stirred solution of 7-(3-carbomethoxyphenyl)-3-phenylmethyl-2,3-dihydrobenzopyran-4-one (6.50 g, 17.5 mmole) in 35 mL of methanol at room temperature was added sodium borohydride (940 mg, 26.0 mmole) in one portion. The dark mixture was stirred at room temperature for 2 hours then poured into saturated aqueous ammonium chloride solution (75 mL) and extracted with 3 times 75 mL of diethyl ether. The combined extracts were washed with brine, dried over sodium sulfate, filtered and concentrated in vacuo to give an off-yellow oil. Chromatography on silica gel eluting with 4:1 hexane: ether afforded first 3.26 g of the cis ring isomer of the title compound, and then 1.98 g of the trans isomer of the title compound as viscous oils, total yield 81%. Cis ring isomer ¹H NMR (300 MHz, CDCl₃): 8.26 (1 H, t, J=1.7 Hz), 8.02 (1 H, dt, J=7.8, 1.7 Hz), 7.76 (1 H, dt, J=7.8, 1.7 Hz), 7.50 (H, t, J=7.8 Hz), 7.41 (1 H, d, J=7.9 Hz), 7.31 (1 H, d, 7.3 Hz), 7.14-7.25 (6 H, m), 4.58 (1 H, t, J=7.2 Hz), 4.28 (1 H, dd, J=9.1, 2.5 Hz), 4.03 (1 H, dd, J=9.1, 5.4 Hz), 3.93 (3 H, s), 2.78 (IH), 2.77 (1 H, dd, J=13.7,6.2 Hz), 2.58 (1 H, dd, J=13.7,9.1 Hz),2.20-2.29 (1 H, m), 1.83 (1 H, d, J=7.2 Hz). Trans ring isomer ¹H NMR (300 MHz, CDCl₃): 8.23 (1 H, t, J=1.7 Hz), 7.98 (1 H, dt, J=7.8 Hz), 7.74 (1 H, t, J=7.8 Hz, 1.7 Hz), 7.48 (1 H, t, J=7.8 Hz), 7.20-7.36 (6 H, m), 7.15 (1 H, dd, J=8.0, 1.8 Hz), 7.09 (1 H, d, J=1.8 Hz), 4.56 (1 H, dt, J=4.7, 3.8 Hz), 4.12-4.19 (2 H, m), 3.92 (3 H, s), 2.90 (1 H, dd, J=13.6, 8.4. Hz), 2.70 (1 H, dd, J=13.6, 7.2 Hz), 2.36-2.39 (1 H, m), 1.75 (1 H, d, J=4.7 Hz).

### I. N-α-t-Butoxycarbonyl-L-tryptophan-7-[(3-carbomethoxyphenyl)-3-phenylmethyl]-2,3-dihydro-benzopyran-4-yl]-ester

To a stirred solution of 7-(3-carbomethoxyphenyl)-4-hydroxy-3-phenylmethyl-2,3-dihydro-benzopyran (2.5 9, 6.7 mmole) in 70 ml of methylene chloride was added DMAP (897 mg., 7.34 mmole, 1.1 equivalents), DCC (1.51 9, 7.34 mmole, 1.1 equivalents) and N-t-Boc-L-tryptophan (2.4 9. 8.01 mmole, 1.2 equivalents). The mixture was stirred at room temperature for 12 hours, filtered and washed with 1 M HCl and brine. The organic layer was dried over MgSO₄, filtered and concentrated in vacuo. Chromatography (silica gel-3:1 cyclohexane:ether) afforded 860 mg of the less polar diastereomer (Rf=0.3) and 700 mg of the more polar moving diastereomer (Rf=0.2). The less polar product (3S, 4R): ¹H-NMR (300 MHz, CDCl₃; 8.29 (1 H, s), 8.03 (2 H, d, J=7.8 Hz), 7.77-7.83 (2 H, m), 7.52 (2 H, t, J=7.6 Hz), 7.02-7.33 (5 H, m), 6.64 (1 H, s), 5.65 (1 H, s), 5.06 (1 H, d, J=8.4 Hz), 4.58-4.62 (1 H, m), 3.95 (3 H. s), 3.73-3.85 (2 H,m), 3.18-3.28 (2 H, m), 2.45-2.61 (2 H, m), 2.09-2.15 (1 H, brd s), 1.39 (9 H, s). The more polar product (3R,4S); ¹H-NMR (300 MHz, CDCl₃): 8.25 (1 H, s), 8.01 (1 H. d, J=7.8 Hz), 7.94 (1 H, brd s), 7.74 (1 H, d, J=8.2 Hz), 7.54 (1 H, d, J=1 1.9 Hz), 7.48 (1 H, t, J=7.8 Hz), 7.09-7.38 (H, m), 6.95 (1 H, s), 5.61 (1 H, s), 5.08 (1 H, d, J=8.2 Hz), 4.554.60 (1 H, m), 3.94 (3 H, s), 3.73-3.76 (2 H, m), 3.22-3.35 (2 H, m), 2.42-2.60 (2 H, m), 1.90-1.96 (1 H, m), 1.39 (9 H, s).

### PREPARATION 5

The following compounds in Table 5 were prepared by saponification in accordance with the procedure described in preparation 4 above.

**TABLE 5**

| R¹ | R³ | Product |
|---|---|---|
| 4-Phenylbenzyl | 5-CI | ¹H-NMR(300 MHz, DMSO_{d6}):7.61-7.67(4H, m), 7.29-7.46 (6H, m), 6.93(1H, brd d, J=7.9Hz), 6.80(1H, br.s.), 4.38(1H, d, J=4.9Hz), 4.16 (1H, brd.d, J=11.0Hz), 4.02 (1H, dd, J=11.0, 5.6Hz), 2.96 (1H, m), 2.56(1 H, m), 2.26 (1H, m). |
| Benzyl | 5-OCH₃ | (cis) ¹H-NMR(300MHz, CDCl₃): 7.96(1H, d, J=8.7Hz), 7.24-7.38(5H, m), 7.16(1H, d, J=8.0Hz), 6.88(1H, dd, J=8.7, 2.6Hz), 6.75-6.83(3H, m), 4.51(1H, d, J=2.9Hz), 4.06-4.15(2H, m), 3.84(3H, s), 2.90(1H, dd, J=13.6, 8.2Hz), 2.70(1H, dd, J=13.6, 7.2Hz), 2.27-2.39(1 H, m). |
| Benzyl | 5-OCH₃ | (trans)¹H-NMR(300MHz, CDCl₃): 7.97(1 H, d, J=8.7Hz), 7.17-7.31(6H, m), 6.85(2H, dt, J=14.3, 2.8Hz), 6.81-6.85 (2H, m), 4.50(1H, d, J=4.1Hz), 4.20(1 H, dd, J=11.2, 2.6Hz), 3.94(1 H, dd,J=11.2, 4.8Hz), 3.86(3H, s), 2.76(1 H, dd, J=13.8, 6.2Hz), 2.52(1H, dd, J=13.2, 9.4Hz), 2.22-2.30(1 H, m). |
| Benzyl | 5-CI | (cis)¹H-NMR(300MHz, CDCl₃): 7.83(1H, d, J=8.4Hz), 7.16-7.38(7H, m), 7.09(1H, d, J=89.1Hz), 6.72-6.84(2H, m), 4.47(1H, d, J=2.8Hz), 4.02-4.12(2H, m), 2.85(1H, dd, J=13.6, 8.1Hz), 2.62(1H, 13.6, 7.4Hz), 2.22-2.38(1H, m). |
| Benzyl | 5-Cl | (trans)¹H-NMR(300MHz, CDCl₃): 7.86(1 H, d, J=8.3Hz), 7.14-7.42(8H, m), 6.76-6.84 (2H, m), 4.48(1H, d, J=4.2Hz), 4.12(1H, dd, J=11.7, 2.6Hz), 3.92(1H, dd, J=11.7, 4.4Hz), 2.73(1H, dd, J=13.7, 6.1Hz), 2.50(1H, dd, J=13.7, 9.5Hz), 2.14-2.26(1H, m). |
| Benzyl | 5-H | (cis)¹H- NMR(300MHz, CDCl₃): 7.88(1 H, dd, J=7.7, 1.2Hz), 7.49(1H, t, J=7.7Hz), 7.11-7.39(8H, m), 6.82-6.89 (2H, m), 4.49(1H, d, J=3.0Hz), 4.06-4.11(2H, m), 2.87(1H, dd, J=13.6, 8.0Hz), 2.63(1H, dd, J=13.6, 7.4Hz), 2.28-2.38(1 H, m). |
| Benzyl | 5-H | (trans) ¹H-NMR(300MHz, CDCl₃): 7.88(1H, dd, J=7.7, 1.2Hz), 7.52(1 H, t, J=7.7Hz), 7.10-7.41 (8H, m), 6.83-6.90 (2H, m), 4.43(1 H, d, J=4.2Hz), 4.12(1H, dd, J=11.2, 2.9Hz), 3.88(1H, dd, J=11.2, 4.5Hz), 2.75(1H, dd, J=13.7, 5.8Hz), 2.51(1H, dd, J=13.7, 9.5Hz), 2.14-2.25(1H, m), mp. 82-84°C. |
| 4-Phenylbenzyl | 5-F | ¹H-NMR(300MHz, DMSO_{d6}): 7.8(1H, dd), 7.01-7.67 (3H, m), 7.29-7.46(6H m), 6.93(HH, brd, d), 6.80(1H, d) 4.38(1H, d)4.16(1H, brd d), 4.01(1H, dd), 2.96(1 H, m), 2.54(1 H, m), 2.22(1 H, m). |

### PPEPARATION 6

By saponification procedure of the corresponding ester in accordance with preparation 4, 7-(4-hydroxy-3-carboxyphenyl)-4-hydroxy-3-phenylmethyl-3,4-dihydro-2H-1-benzopyran was formed having a melting point of 158-160°C. (cis) and 173-175°C. (trans).

While the invention has been described and illustrated with reference to certain particular embodiments thereof, those skilled in the art will appreciate that various adaptations, changes, modifications, substitutions, deletions, or additions of procedures and protocols may be made without departing from the spirit and scope of the invention. It is intended, therefore, that the invention be defined by the scope of the claims that follow and that such claims be interpreted as broadly as is reasonable.

## Claims

1. The use of an LTB₄ antagonist agent for the preparation of a medicament for the treament of atherosclerosis in a mammalian subject.

2. The use according to claim 1, wherein said LTB₄ antagonist agent is selected from the group consisting of an antibody for the LTB₄ receptor and an antisense for the LTB₄ gene.

3. The use according to claim 1, wherein said LTB₄ antagonist agent exhibits an LTB₄ IC₅₀ of less than about 20 nM as measured by chemotaxis assay.

4. The use according to claim 1, wherein said LTB4 antagonist agent is a small molecule.

5. The use according to claim 4, wherein said small molecule exhibits an LTB₄ IC₅₀ of less than about 20 nM as measured by chemotaxis assay.

6. The use according to claim 1, wherein the LTB₄ antagonist agent is a compound of the formula 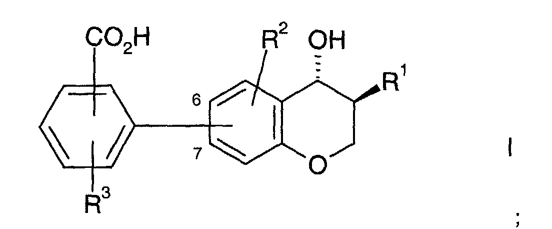 an enantiomer thereof; or a pharmaceutically acceptable salt thereof; effective to treat atherosclerosis;
wherein in said compound of formula I the R³-substituted benzoic acid moiety is attached at carbon 6 or 7 of the benzopyran ring;
R¹ is -(CH₂)_{q}CHR⁵R⁶, wherein q is 0 to 4;
each R² and R³ is independently selected from the group consisting of hydrogen, fluoro, chloro, (C₁-C₆)alkyl, (C₁-C₆)alkyloxy, phenylsulfinyl, phenylsulfonyl, and (C₁-C₆)alkyl-S(O)ₙ-, wherein said n is 0 to 2; wherein said R² and R³ alkyl moiety wherever they occur are independently optionally substituted by one to three fluoro groups; and wherein said R² and R³ phenyl moiety wherever they occur are independently optionally substituted by one to three fluoro groups;
R⁵ is hydrogen, (C₁-C₆)alkyl, or phenyl;
wherein said R⁵ phenyl is optionally substituted by fluoro, chloro, (C₁-C₆)alkyl, (C₁-C₆)alkyloxy, phenylsulfinyl, phenylsulfonyl, or (C₁-C₆)alkyl-S(O)ₙ-, wherein said n is 0 to 2; and wherein said alkyl moiety wherever they occur on said phenyl optional substituent are independently optionally substituted by one to three fluoro groups; and wherein said phenyl wherever they occur on said phenyl optional substituent are independently optionally substituted by one to three fluoro groups;
R⁶ is hydrogen, (C₁-C₆)alkyl, (C₃-C₈)cycloalkyl, phenyl, or 5 to 10 membered heteroaryl;
wherein said R⁶ phenyl is optionally substituted by fluoro, chloro, (C₁-C₆)alkyl, (C₁-C₆)alkyloxy, phenylsulfinyl, phenylsulfonyl, or (C₁-C₆)alkyl-S(O)ₙ-, wherein said n is 0 to 2; and wherein said alkyl moiety wherever they occur on said phenyl optional substituent are independently optionally substituted by one to three fluoro groups; and wherein said phenyl moiety wherever they occur on said phenyl optional substituent are independently optionally substituted by one to three fluoro groups; and
wherein said 5 to 10 membered heteroaryl is optionally substituted by 1 or 2 substituents independently selected from fluoro, chloro, (C₁-C₆)alkyl, (C₁-C₆)alkyloxy, phenylsulfinyl, phenylsulfonyl, or (C₁-C₆)alkyl-S(O)ₙ-, wherein said n is 0 to 2; wherein said alkyl moiety wherever they occur on said 5 to 10 membered heteroaryl optional substituent are independently optionally substituted by one to three fluoro groups; and wherein said phenyl moiety wherever they occur on said 5 to 10 membered heteroaryl optional substituent are independently optionally substituted by one to three fluoro groups.

7. The use according to claim 6, wherein, in the LTB₄ antagonist agent of formula I, R¹ is benzyl, 4-fluorobenzyl, 4-phenylbenzyl, 4-(4-fluorophenyl)benzyl, or phenethyl.

8. The use according to claim 6 or claim 7, wherein, in the LTB₄ antagonist agent of formula I, R² is hydrogen or fluoro.

9. The use according to any of claims 6 to 8, wherein, in the LTB₄ antagonist agent of formula I, the R³-substituted benzoic acid moiety is attached at carbon 7 of the benzopyran ring and wherein the R³-substituted benzoic acid moiety is 2-carboxyphenyl, 2-carboxy-5-chlorophenyl, 2-carboxy-4-chlorophenyl, 2-carboxy-3-fluorophenyl, 2-carboxy-5-fluorophenyl, 2-carboxy-5-trifluoromethylphenyl, 2-carboxy-4-fluorophenyl, 2-carboxy-6-fluorophenyl, or 3-carboxyphenyl.

10. The use according to any of claims 6 to 9, wherein, in the LTB₄ antagonist agent of formula I, R¹ is benzyl, R² is hydrogen, and the R³-substituted benzoic acid moiety is 2-carboxy-5-fluorophenyl.

11. The use according to any of claims 6 to 9, wherein, in the LTB₄ antagonist agent of formula I, R¹ is 4-phenylbenzyl, R² is hydrogen, and the R³-substituted benzoic acid moiety is 2-carboxy-5-fluorophenyl or 2-carboxy-4-chlorophenyl.

12. The use according to any of claims 6 to 10, wherein the LTB₄ antagonist agent of formula I is (3S,4R)-7-(2-carboxy-5-trifluoromethylphenyl)-4-hydroxy-3-benzyl-3,4-dihydro-2H-1-benzopyran.

13. The use according to any of claims 6 to 12, wherein the LTB₄ antagonist agent of formula I is impregnated in a stent.

14. The use according to claim 13, wherein the LTB₄ antagonist agent of formula I is (3S, 4R)-7-(2-carboxy-5-trifluoromethylphenyl)-4-hydroxy-3-benzyl-3,4-dihydro-2H-1-benzopyran.
